# EUROPEAN PATENT APPLICATION

(11) **EP 4 613 250 A1**
(43) Date of publication of application: **10.09.2025**
(21) Application number: 24744593.5
(22) Date of filing: 12.01.2024
(51) Int. Cl.: A61F 13/15

(54) **STRETCHABLE SHEET MANUFACTURING METHOD, STRETCHABLE SHEET, STRETCHABLE SHEET MANUFACTURING DEVICE, AND ANVIL ROLL**

(30) Priority: 20.01.2023 JP 2023007690
(71) Applicant: Unicharm Corporation, Ehime 799-0111 (JP)
(72) Inventor: NOMA, Shinji, Kanonji-shi, Kagawa 769-1602 (JP); MUKAI, Hirotomo, Kanonji-shi, Kagawa 769-1602 (JP); BABA, Ryo, Kanonji-shi, Kagawa 769-1602 (JP); NARA, Yuki, Kanonji-shi, Kagawa 769-1602 (JP); NAGAI, Takahito, Kanonji-shi, Kagawa 769-1602 (JP); TANAKA, Suguru, Kanonji-shi, Kagawa 769-1602 (JP)
(74) Representative: Dolleymores
(86) International application number: PCT/JP2024/000664
(87) International publication number: WO 2024/154670

(57) **Abstract**

Provided is a stretchable sheet manufacturing method including: a step for supplying first and second sheets (11, 12) and an elastic member (14); and a bonding step for forming a pair of welds (jP) on both sides of the elastic member (14) and bonding the first and second sheets (11, 12). A plurality of protruding sections (231) of an anvil roll (23) have a groove section (232), and in the intersecting direction cross-section of the groove section (232), when intersection points between a line (X₁) connecting starting edges (232s) of first side wall sections (232iu) of the groove section (232) and lines (X₂) along which second side wall sections (232id) extend toward the respective starting edge (232s) sides are denoted as first and second intersection points (Y₁, Y₂), the separation distance (W1) between the starting edges (232s) is longer than the distance (W2) between the first and second intersection points (Y₁, Y₂).

## Description

### FIELD

The present invention relates to a method for manufacturing a stretchy sheet, a stretchy sheet, a unit for manufacturing a stretchy sheet, and an anvil roll.

### BACKGROUND

As a method for manufacturing a stretchy sheet, there is known a method of attaching an elastic member (e.g., elastic string) in a stretched state to between sheets, by the elastic member being sandwiched between welded portions. For example, Patent Document 1 discloses a method for forming the following porosity change region: in a stretchy sheet which has an elastic member in a stretched state being placed between a first sheet and a second sheet, and in which the first sheet and the second sheet are joined by a plurality of fusion parts located on both sides of the elastic member, the porosity change region is region in which a part of the constituent fibers is melted between a pair of fusion parts of the second sheet.

### [CITATION LIST]

### [PATENT LITERATURE]

[Patent Document 1] Japanese Patent No. 6997254

### SUMMARY

### [TECHNICAL PROBLEM]

In the manufacturing method disclosed in Patent Document 1, the porosity change region is formed by fusion using ultrasonic fusion parts, and when a stacked body is supplied to the ultrasonic fusion part, the depth and width of the depressed portion that contains the stacked body are adjusted so that voids do not exist between the first sheet, the elastic member, and the second sheet in the stacked body. However, with the adjustment of the depressed portion in Patent Document 1, in cases where an elastic member having multiple diameters are placed in the stretchy sheet, there is no room in the depressed portion, causing a risk that the elastic member will protrude or come off. In addition, when manufacturing a stretchy sheet by sandwiching an elastic member (e.g., elastic string) between welded portions, if the width between the welded portions where the elastic member is sandwiched is narrowed in order to securely hold it in place, the elastic member may break when forming the welded portions. On the other hand, if the width between the welded portions where the elastic member is sandwiched is widened in order not to cut the elastic member, there is a risk the elastic member will come off during manufacturing. Accordingly, during a process for manufacturing a stretchy sheet, it has been difficult to attach a plurality of elastic members without cutting them.

The present invention is achieved in light of conventional problems such as that described above, and an aspect of the present invention is, in a method for manufacturing a stretchy sheet in which an elastic member is attached to a sheet by sandwiching the elastic member between the welded-portion pair located on both sides of the elastic member, to prevent the elastic member from being cut in the joining step in which the welded-portion pair is formed and to attach an elastic member so that it does not come off.

### [SOLUTION TO PROBLEM]

A main aspect of the present invention of achieving the above-described aspect is a method for manufacturing a stretchy sheet in which a first sheet and a second sheet are stacked with an elastic member in between, the method including: a step of supplying the first sheet, the second sheet, and the elastic member to an anvil roll that rotates in a direction of transport, the elastic member being in a state of stretching in the direction of transport and being placed between the first sheet and the second sheet; a joining step of joining the first sheet and the second sheet by forming a welded-portion pair on both sides, in an intersecting direction perpendicular to the direction of transport, of the elastic member; and a step of cutting the elastic member, wherein after the step of cutting, the elastic member that has contracted is sandwiched between the welded-portion pair, and a position, in the direction of transport, of the elastic member is restricted, wherein an outer circumferential surface of the anvil roll has a plurality of projection portions that project outside in a radial direction of the anvil roll, wherein the projection portions have a groove portion along a rotational direction of the anvil roll, wherein, in a cross section of the groove portion, taken along the intersecting direction, the groove portion has a pair of first side-wall portions extending from a starting edge of the groove portion to a specified position on a far side, and a pair of second side-wall portions extending from the specified position to a bottom portion of the groove portion, wherein, in the cross section, when intersection points of a line connecting starting edges of the pair of first side-wall portions in the intersecting direction and lines respectively extending the pair of second side-wall portions toward the starting edges are a first intersection point and a second intersection point, a separation distance between the starting edges of the pair of first side-wall portions is longer than a distance between the first intersection point and the second intersection point.

Features of the present invention other than the above will become clear by reading the description of the present specification with reference to the accompanying drawings.

### [ADVANTAGEOUS EFFECT OF THE INVENTION]

According to the present invention, in a method for manufacturing a stretchy sheet in which an elastic member is attached to a sheet by sandwiching the elastic member between the welded-portion pair located on both sides of the elastic member, it is possible to prevent the elastic member from being cut in the joining step in which the welded-portion pair is formed and to attach an elastic member so that it does not come off.

### BRIEF DESCRIPTION OF DRAWINGS

FIG. 1 is a diagram of a stretchy sheet 10 in a state of being stretched to eliminate wrinkles.
FIG. 2 is a schematic cross-sectional view in a portion indicated by symbol A in FIG. 1.
FIG. 3 is an enlarged view of a portion indicated by symbol B in FIG. 1.
FIG. 4A is a schematic perspective view of an absorbent article 1 in which the stretchy sheet 10 is used, and FIG. 4B is a schematic plan view of the absorbent article 1 in an unfolded state and a stretched state, viewed from the skin-side face side.
FIG. 5 is a schematic cross-sectional view of a manufacturing apparatus 20 for a stretchy sheet in the present embodiment.
FIG. 6 is a schematic cross-sectional view of an ultrasonic welding unit 22.
FIG. 7A is a diagram illustrating the surface of an anvil roll 23. FIG. 7B is a schematic enlarged view of the nearest neighbors of the anvil roll 23 and a horn 24.
FIGS. 8A and 8B are diagrams illustrating a method for attaching an elastic member 14 by welded portions j.
FIG. 9 is a cross-sectional view showing a cross section of a groove portion 232, taken along the intersecting direction.
FIG. 10 is a diagram illustrating the configuration of the groove portion 232.
FIGS. 11A to 11C are diagrams showing modified examples of the groove portion 232.
FIG. 12 is a perspective view illustrating a projection portion 231.
FIG. 13 is a diagram illustrating a joining step in a state where a first elastic member 14a and a second elastic member 14b are interposed.
FIG. 14A is a schematic cross-sectional view of a portion of the stretchy sheet 10 where a welded-portion pair jP is formed, in a natural state.
FIG. 14B is a schematic cross-sectional view of another example of a portion of the stretchy sheet 10 where the welded-portion pair jP is formed, in the natural state.
FIG. 14C is a microscope image showing an example of an inner welded portion jin and an outer welded portion jout according to the present embodiment.
FIG. 14D is a microscope image showing another example of the inner welded portion jin and the outer welded portion jout according to the present embodiment.
FIG. 15 is a diagram illustrating the structure of the elastic member 14.
FIG. 16A shows a modified example of the anvil roll 23, and FIG. 16B is a diagram illustrating groove portions 232 of the anvil roll 23 in FIG. 16A.
FIG. 17 shows a modified example of the stretchy sheet 10, and is a diagram of the stretchy sheet 10 in a state of being stretched to eliminate wrinkles.

### DESCRIPTION OF EMBODIMENTS

At least the following matters will be clear with the description of this specification and the attached drawings

### Aspect 1:

A method for manufacturing a stretchy sheet in which a first sheet and a second sheet are stacked with an elastic member in between, the method including: a step of supplying the first sheet, the second sheet, and the elastic member to an anvil roll that rotates in a direction of transport, the elastic member being in a state of stretching in the direction of transport and being placed between the first sheet and the second sheet; a joining step of joining the first sheet and the second sheet by forming a welded-portion pair on both sides, in an intersecting direction perpendicular to the direction of transport, of the elastic member; and a step of cutting the elastic member, wherein after the step of cutting, the elastic member that has contracted is sandwiched between the welded-portion pair, and a position, in the direction of transport, of the elastic member is restricted, wherein an outer circumferential surface of the anvil roll has a plurality of projection portions that project outside in a radial direction of the anvil roll, wherein the projection portions have a groove portion along a rotational direction of the anvil roll, wherein, in a cross section of the groove portion, taken along the intersecting direction, the groove portion has a pair of first side-wall portions extending from a starting edge of the groove portion to a specified position on a far side, and a pair of second side-wall portions extending from the specified position to a bottom portion of the groove portion, wherein, in the cross section, when intersection points of a line connecting starting edges of the pair of first side-wall portions in the intersecting direction and lines respectively extending the pair of second side-wall portions toward the starting edges are a first intersection point and a second intersection point, a separation distance between the starting edges of the pair of first side-wall portions is longer than a distance between the first intersection point and the second intersection point.

According to the method for manufacturing a stretchy sheet of Aspect 1, the separation distance between the starting edges of the first side-wall portions defines the opening width of the groove portion, and the groove portion is configured such that a groove width is wider toward the opening from a specified position on the far side with respect to the opening. With this configuration, the elastic member is placed in the joining step without protruding from the groove portion, making it easier to join the first sheet and the second sheet. By preventing the elastic member from being welded unintendedly in the joining step, it is possible to prevent the elastic member from being cut, and by carrying out supplying and joining steps while steadily passing the elastic member through the groove portion, it is possible to prevent the elastic member from coming off during the steps.

### Aspect 2:

A method for manufacturing a stretchy sheet in which a first sheet and a second sheet are stacked with an elastic member in between, the method including: a step of supplying the first sheet, the second sheet, and the elastic member to an anvil roll that rotates in a direction of transport, the elastic member being in a state of stretching in the direction of transport and being placed between the first sheet and the second sheet; a joining step of joining the first sheet and the second sheet by forming a welded-portion pair on both sides, in an intersecting direction perpendicular to the direction of transport, of the elastic member; and a step of cutting the elastic member, wherein after the step of cutting, the elastic member that has contracted is sandwiched between the welded-portion pair, and a position, in the direction of transport, of the elastic member is restricted, wherein an outer circumferential surface of the anvil roll has a plurality of projection portions that project outside in a radial direction of the anvil roll, wherein the projection portions have a groove portion along a rotational direction of the anvil roll, wherein, in a cross section of the groove portion, taken along the intersecting direction, a depth of the groove portion is equal to or greater than a height of the projection portion from the outer circumferential surface, and a length, in the intersecting direction, of the groove portion is shorter from the starting edge of the groove portion toward a far side.

According to the method for manufacturing a stretchy sheet of Aspect 2, in cases where the groove portion is shallow, there is a risk that the elastic member will protrude and be cut off during the joining step, but in cases where the groove portion is deep enough, the elastic member can pass stably through the groove portion. In addition, the length in the intersecting direction (width) becomes shorter toward the far side in the groove portion, making it more difficult for the elastic member to come out from the groove portion. This allows the joining step to be performed without cutting off the elastic member.

### Aspect 3:

The method for manufacturing a stretchy sheet according to Aspect 1 or 2, wherein the projection portions have a plurality of the groove portions for the elastic member, the plurality of groove portions being located spaced apart in the intersecting direction.

According to the method for manufacturing a stretchy sheet of Aspect 3, by providing multiple groove portions in the projection portion, even if the placement of the elastic member is slightly shifted during manufacturing, as long as the elastic member is placed in any of the multiple groove portions, the joining step can be carried out without any cutting or coming off.

### Aspect 4:

The method for manufacturing a stretchy sheet according to Aspect 3, wherein, in the cross section, a depth of the groove portions is greater than or equal to a spacing of the groove portions in the intersecting direction.

According to the method for manufacturing a stretchy sheet of Aspect 4, since the depth of the groove portion is large, the elastic member can pass more stably through the groove portion.

### Aspect 5:

The method for manufacturing a stretchy sheet according to Aspect 1, wherein, in the cross section, a length, in the intersecting direction, of the groove portion is shorter from the starting edge of the groove portion toward a far side.

According to the method for manufacturing a stretchy sheet of Aspect 5, this shape of the groove portion makes it easier for the elastic member to enter the groove portion and less likely to come out, making it less likely for the elastic member to be cut off or damaged during the supplying and joining step.

### Aspect 6:

The method for manufacturing a stretchy sheet according to Aspect 1, wherein, in the cross section, an angle formed by the pair of first side-wall portions is equal to or greater than twice an angle formed by the pair of second side-wall portions.

According to the method for manufacturing a stretchy sheet of Aspect 6, this shape of the groove portion makes it easier for the elastic member to enter the groove portion and less likely to come out, making it less likely for the elastic member to be cut off or damaged during the supplying and joining step.

### Aspect 7:

The method for manufacturing a stretchy sheet according to Aspect 7, wherein, in a depth direction of the groove portion in the cross section, a length of a portion, of the groove portion, formed by the pair of second side-wall portions is longer than a length of a portion, of the groove portion, formed by the pair of first side-wall portions.

According to the method for manufacturing a stretchy sheet of Aspect 7, because the length, on the far side (bottom portion side), of the groove portion, is long, it becomes easier to accommodate the elastic member up to the far side of the groove portion when passing the elastic member through the groove portion, making it less likely for the elastic member to come out of the groove portion.

### Aspect 8:

The method for manufacturing a stretchy sheet according to any one of Aspects 1 to 7, wherein a depth of the groove portion in the cross section is greater than an outer diameter of the elastic member when the elastic member is stretched to its maximum stretchable length in the direction of transport.

According to the method for manufacturing a stretchy sheet of Aspect 8, This makes it difficult for the elastic member placed in the stretched state to come out of the groove portion during the manufacturing steps, and allows the joining step to be carried out without cutting the elastic member.

### Aspect 9:

The method for manufacturing a stretchy sheet according to Aspect 8, wherein a depth of the groove portion in the cross section is greater than a maximum outer diameter of the elastic member which has contracted after the cutting step.

According to the method for manufacturing a stretchy sheet of Aspect 9, by making the groove portion have a sufficient depth, the elastic member is less likely to come out of the groove portion, making it possible to carry out the joining step without cutting the elastic member.

### Aspect 10:

The method for manufacturing a stretchy sheet according to any one of Aspects 1 to 6, 8 and 9, wherein, in the cross section, an area ratio occupied by a portion of the groove portion that corresponds to the pair of first side-wall portions, in the depth direction of the groove portion, is larger than an area ratio occupied by a portion of the groove portion that corresponds to the pair of second side-wall portions.

According to the method for manufacturing a stretchy sheet of Aspect 10, since the groove portion has a configuration in which the first side-wall portion side (starting edge side) is large, it becomes easier for the elastic member to enter the groove portion, making it possible to place the elastic member stably.

### Aspect 11:

The method for manufacturing a stretchy sheet according to Aspect 2, wherein, in a cross section of the groove portion, taken along the intersecting direction, the groove portion has a pair of first side-wall portions extending from a starting edge of the groove portion to a specified position on a far side, and a pair of second side-wall portions extending from the specified position to a bottom portion of the groove portion, and in the cross section, when intersection points of a line connecting starting edges of the pair of first side-wall portions in the intersecting direction and lines respectively extending the pair of second side-wall portions toward the starting edges are a first intersection point and a second intersection point, a separation distance between the starting edges of the pair of first side-wall portions is longer than a distance between the first intersection point and the second intersection point.

According to the method for manufacturing a stretchy sheet of Aspect 11, not only does the groove portion have sufficient depth, but the opening width of the groove portion is wide, making it easier for the elastic member to be placed without protruding from the groove portion. Therefore, the elastic member is more likely to tightly stay within the groove portion, and the elastic member is less susceptible to the influence of the projection portion.

### Aspect 12:

A method for manufacturing a stretchy sheet in which a first sheet and a second sheet are stacked with a plurality of elastic members in between, the method including: a step of supplying the first sheet, the second sheet, and the plurality of elastic members to an anvil roll that rotates in a direction of transport, the plurality of elastic members being in a state of stretching in the direction of transport and a state of being placed between the first sheet and the second sheet, with spaced apart in an intersecting direction that is perpendicular to the direction of transport; a joining step of joining the first sheet and the second sheet by forming a welded-portion pair on both sides, in the intersecting direction perpendicular to the direction of transport, of each of the elastic members; and a step of cutting the elastic member, wherein the plurality of elastic members supplied to the anvil roll in the supplying step include a first elastic member and a second elastic member having different thicknesses, wherein, in the joining step, the welded-portion pair is formed for each of the first elastic member and the second elastic member so that a damaged portion that a part of a surface is damaged is not formed, and wherein in the joining step, the welded-portion pair is formed in such a manner that the first elastic member and the second elastic member that have contracted after the step of cutting are each sandwiched between the welded-portion pair corresponding to it so that positions, in the direction of transport, of the first elastic member and the second elastic member are restricted.

According to the method for manufacturing a stretchy sheet of Aspect 12, even with the first elastic member and the second elastic member, which have different thicknesses in the stretched state and different stretch factors, the joining step can be performed without the damaged portion occurring on the surface of both, and it is possible to restrict their positions in the direction of transport without coming off.

### Aspect 13:

The method for manufacturing a stretchy sheet according to Aspect 12, wherein an outer circumferential surface of the anvil roll has a plurality of projection portions that project outside in a radial direction of the anvil roll, the plurality of projection portions have a groove portion along a rotational direction of the anvil roll, and, in the joining step, the welded-portion pair is formed without the first elastic member and the second elastic member coming out of the groove portions in which they are respectively placed.

According to the method for manufacturing a stretchy sheet of Aspect 13, even with the first elastic member and the second elastic member that have different thicknesses in the stretched state, the welded-portion pair is formed without the elastic members coming out of the groove portion, and therefore any of the elastic members can be fixed in place without the welded-portion pair being placed thereon (cutting them).

### Aspect 14:

The method for manufacturing a stretchy sheet according to Aspect 12 or 13, wherein the projection portions have a plurality of the groove portions for a single one of the elastic members, the plurality of groove portions being located spaced apart in the intersecting direction.

According to the method for manufacturing a stretchy sheet of Aspect 14, by the projection portion having multiple groove portions for a single one of the elastic members, even if the placement of the elastic member is slightly shifted during manufacturing, as long as the elastic member is placed in any of the multiple groove portions, the joining step can be carried out without any cutting or coming off.

### Aspect 15:

The method for manufacturing a stretchy sheet according to any one of Aspects 12 to 14, wherein, in a cross section of the groove portion taken along the intersecting direction, a depth of the groove portions is greater than or equal to a spacing of the groove portions in the intersecting direction.

According to the method for manufacturing a stretchy sheet of Aspect 15, since the depth of the groove portion is large, even if multiple elastic members with different thicknesses are placed in the stretched state, they can pass through the groove portion more stably.

### Aspect 16:

The method for manufacturing a stretchy sheet according to any one of Aspects 12 to 15, wherein, in a cross section of the groove portion taken along the intersecting direction, a depth of the groove portion is greater than an outer diameter of each of the first elastic member and the second elastic member when each of the first elastic member and the second elastic member is stretched to its maximum stretchable length in the direction of transport.

According to the method for manufacturing a stretchy sheet of Aspect 16, even if there is variation in the stretched state between the first elastic member and the second elastic member, which have different thicknesses, the above-mentioned configuration allows the step to be carried out in which the elastic member pass stably through the groove portion.

### Aspect 17:

The method for manufacturing a stretchy sheet according to any one of Aspects 12 to 16, wherein a depth of the groove portion in the cross section is greater than a maximum outer diameter of each of the first elastic member and the second elastic member which have contracted after the cutting step.

According to the method for manufacturing a stretchy sheet of Aspect 17, by making the groove portion have a sufficient depth, the the first elastic member and the second elastic member are less likely to come out of the groove portion, making it possible to carry out the joining step without cutting the elastic member.

### Aspect 18:

The method for manufacturing a stretchy sheet according to any one of Aspects 12 to 17, wherein an outer circumferential surface of the anvil roll has a plurality of projection portions that project outside in a radial direction of the anvil roll, the projection portions have a groove portion along a rotational direction of the anvil roll, in a cross section of the groove portion taken along the intersecting direction, the groove portion has a pair of first side-wall portions extending from a starting edge of the groove portion to a specified position on a far side, and a pair of second side-wall portions extending from the specified position to a bottom portion of the groove portion, and in the cross section, an area ratio occupied by a portion of the groove portion that corresponds to the pair of first side-wall portions, in the depth direction of the groove portion, is larger than an area ratio occupied by a portion of the groove portion that corresponds to the pair of second side-wall portions.

According to the method for manufacturing a stretchy sheet of Aspect 18, even if the plurality of elastic members having different thicknesses in the stretched state are arranged, when passing the thick elastic member through the groove portion while receiving the elastic member into the groove portion on the starting edge side, adjustments can be made at the groove portion on the far side (on the bottom portion side). This makes it possible to carry out the joining step more stably.

### Aspect 19:

A stretchy sheet having an up-down direction and a left-right direction that intersect each other, the stretchy sheet including: a first sheet; a second sheet that is joined to the first sheet by a plurality of welded portions; and an elastic member that is provided between the first sheet and the second sheet and that is capable of stretching/contracting in the left-right direction, wherein a plurality of welded-portion pairs are included in which the plurality of welded portions are located on both sides, in the up-down direction, of the elastic member, and that restricts a position, in the left-right direction, of the elastic member with respect to the first sheet and the second sheet, by sandwiching the elastic member in a state of being contracted in the left-right direction, wherein, in a cross section of the stretchy sheet taken along the up-down direction at a formation position of the welded portion, the welded-portion pair is provided on both sides, in the up-down direction, of the elastic member, and at least either one welded portion of the welded-portion pair has an inner welded portion that includes fibers having a melted-and-solidified portion in which constituent fibers constituting the first sheet and the second sheet have been melted and solidified, and an outer welded portion that is located outside the inner welded portion in the up-down direction and that includes only fibers having the melted-and-solidified portion, and wherein an average thickness of the inner welded portion is larger than an average thickness of the outer welded portion.

According to the stretchy sheet of Aspect 19, by the welded portion having the inner welded portion, the welded portion is prevented from becoming too stiff, and in addition, even if force is applied to the inner welded portion and the positional restriction of the elastic member weakens, the presence of the outer welded portion prevents the elastic member from coming out from between the first sheet and the second sheet. In addition, the thickness of the inner welded portion makes it easier to cushion the force applied to the welded portion, preventing the outer welded portion from tearing or peeling off.

### Aspect 20:

The stretchy sheet according to Aspect 19, wherein in a cross section of the stretchy sheet taken along the up-down direction, both welded portions of the welded-portion pair have the inner welded portion and the outer welded portion.

According to the stretchy sheet of Aspect 19, on both sides of the elastic member in the up-down direction, it is possible to prevent the welded portion from becoming too stiff, while preventing the elastic member from coming out from between the first sheet and the second sheet.

### Aspect 21:

The stretchy sheet according to Aspect 19 or 20, wherein n a cross section of the stretchy sheet taken along the up-down direction, the stretchy sheet has a portion where a void is formed at least either between the elastic member and the first sheet or between the elastic member and the second sheet.

According to the stretchy sheet of Aspect 21, by placing the elastic member while forming the void, without excessive contact with the first sheet and the second sheet, it prevents the elastic member sandwiched between the welded-portion pair from becoming too stiff when the stretchy sheet contracts.

### Aspect 22:

The stretchy sheet according to any one of Aspects 19 to 21, wherein the elastic member is a string-like elastic member made of a plurality of elastic fibers, and the elastic member has a void between the elastic fibers, in a cross section taken along the up-down direction between the welded-portion pair between which the elastic member in a state of contracting in the left-right direction is sandwiched.

According to the stretchy sheet of Aspect 22, even if external pressure is applied to the elastic member, the void serves as an escape space by being compressed, making it difficult for the elastic member to be cut off.

### Aspect 23:

An absorbent article having an up-down direction and a left-right direction that intersect each other, the absorbent article including: an absorbent main body; and a pair of waist portions, wherein the absorbent article includes a first sheet, a second sheet that is joined to the first sheet by a plurality of welded portions, and an elastic member that is capable of stretching/contracting in the left-right direction, wherein a plurality of the elastic members are located at intervals in the up-down direction between the first sheet and the second sheet, wherein the plurality of welded portions includes a plurality of welded-portion pairs located on both sides, in the up-down direction, of the elastic member, wherein the plurality of welded-portion pairs restricts a position, in the left-right direction, of the elastic member with respect to the first sheet and the second sheet, by sandwiching the elastic member in a state of being contracted in the left-right direction, wherein the plurality of elastic members include a first elastic member and a second elastic member having different thicknesses in a state where the absorbent article is maximally stretched in the left-right direction, and wherein, for each of the first elastic member and the second elastic member, a damaged portion in which a part of a surface of each of the first elastic member and the second elastic member is damaged is not provided between the welded-portion pairs adjacent in the left-right direction.

According to the absorbent article of Aspect 23, by having the first elastic member and the second elastic member, which have different thicknesses when stretched, between the first sheet and the second sheet, it is possible to provide parts having different stretch factors, allowing each part of the absorbent article to exhibit the desired stress or stretch characteristics.

### Aspect 24:

An apparatus for manufacturing a stretchy sheet in which is a first sheet and a second sheet are stacked with an elastic member in between, the apparatus, including: a supplying section that supplies the first sheet, the second sheet, and the elastic member to an anvil roll that rotates in a direction of transport, the elastic member being in a state of stretching in the direction of transport and being placed between the first sheet and the second sheet; a welded-portion forming section that forms a plurality of welded portions that join the first sheet and the second sheet, by forming a welded-portion pair on both sides, in an intersecting direction perpendicular to the direction of transport, of the elastic member; and a cutting section that cuts the elastic member; wherein after the step of cutting, the elastic member that has contracted is sandwiched between the welded-portion pair, and a position, in the direction of transport, of the elastic member is restricted, wherein an outer circumferential surface of the anvil roll has a plurality of projection portions that project outside in a radial direction of the anvil roll, wherein the projection portions have a groove portion along a rotational direction of the anvil roll, wherein, in a cross section of the groove portion, taken along the intersecting direction, the groove portion has a pair of first side-wall portions extending from a starting edge of the groove portion to a specified position on a far side, and a pair of second side-wall portions extending from the specified position to a bottom portion of the groove portion, wherein, in the cross section, when intersection points of a line connecting starting edges of the pair of first side-wall portions in the intersecting direction and lines respectively extending the pair of second side-wall portions toward the starting edges are a first intersection point and a second intersection point, a separation distance between the starting edges of the pair of first side-wall portions is longer than a distance between the first intersection point and the second intersection point.

According to the apparatus for manufacturing a stretchy sheet of Aspect 24, the separation distance between the starting edges of the first side-wall portions defines the opening width of the groove portion, and the groove portion is configured such that a groove width is wider toward the opening from a specified position on the far side with respect to the opening. With the apparatus having this configuration, the elastic member is placed without protruding from the groove portion, making it easier to join the first sheet and the second sheet. By preventing the elastic member from being welded unintendedly, it is possible to prevent the elastic member from being cut, and by carrying out the manufacturing steps while steadily passing the elastic member through the groove portion, it is possible to prevent the elastic member from coming off during manufacturing.

### Aspect 25:

An apparatus for manufacturing a stretchy sheet in which is a first sheet and a second sheet are stacked with an elastic member in between, the apparatus, including: a supplying section that supplies the first sheet, the second sheet, and the elastic member to an anvil roll that rotates in a direction of transport, the elastic member being in a state of stretching in the direction of transport and being placed between the first sheet and the second sheet; a welded-portion forming section that forms a plurality of welded portions that join the first sheet and the second sheet, by forming a welded-portion pair on both sides, in an intersecting direction perpendicular to the direction of transport, of the elastic member; and a cutting section that cuts the elastic member; wherein after the step of cutting, the elastic member that has contracted is sandwiched between the welded-portion pair, and a position, in the direction of transport, of the elastic member is restricted, wherein an outer circumferential surface of the anvil roll has a plurality of projection portions that project outside in a radial direction of the anvil roll, wherein the projection portions have a groove portion along a rotational direction of the anvil roll, wherein, in a cross section of the groove portion, taken along the intersecting direction, a depth of the groove portion is equal to or greater than a height of the projection portion from the outer circumferential surface, and a length, in the intersecting direction, of the groove portion is shorter from the starting edge of the groove portion toward a far side.

According to the apparatus for manufacturing a stretchy sheet of Aspect 25, in cases where the groove portion is shallow, there is a risk that the elastic member will protrude and be cut off when forming the welded portion. However, with the manufacturing apparatus having the configuration described above, the groove portion has sufficient depth, making it possible for the elastic member to pass stably through the groove portion and to form the welded portion without cutting off the elastic member.

### Aspect 26:

An apparatus for manufacturing a stretchy sheet in which a first sheet and a second sheet are stacked with a plurality of elastic members in between, the apparatus including: a supplying section that supplies the first sheet, the second sheet, and the plurality of elastic members to an anvil roll that rotates in a direction of transport, the plurality of elastic members being in a state of stretching in the direction of transport and a state of being placed between the first sheet and the second sheet, with spaced apart in an intersecting direction that is perpendicular to the direction of transport; a welded-portion forming section that forms a plurality of the welded portions that join the first sheet and the second sheet, by forming a welded-portion pair on both sides, in the intersecting direction perpendicular to the direction of transport, of each of the elastic members; and a cutting section that cuts the elastic member, wherein the plurality of elastic members supplied to the anvil roll in the supplying section include a first elastic member and a second elastic member having different thicknesses, wherein, in the welded-portion forming section, the welded-portion pair is formed for each of the first elastic member and the second elastic member so that a damaged portion that a part of a surface is damaged is not formed, and wherein in the welded-portion forming section, the welded-portion pair is formed in such a manner that the first elastic member and the second elastic member that have contracted after cutting by the cutting section are each sandwiched between the welded-portion pair corresponding to it so that positions, in the direction of transport, of the first elastic member and the second elastic member are restricted.

According to the apparatus for manufacturing a stretchy sheet of Aspect 26, even with the first elastic member and the second elastic member, which have different thicknesses in the stretched state and different stretch factors, the joining step can be performed without the damaged portion occurring on the surface of both, and it is possible to restrict their positions, in the direction of transport, of the elastic members without the elastic members coming off.

### Aspect 27:

An anvil roll that uses for an apparatus for manufacturing a stretchy sheet in which a first sheet and a second sheet are stacked with the elastic member in between, the anvil roll including: a plurality of projection portions that project outside in a radial direction of the anvil roll, on an outer circumferential surface of the anvil roll, wherein the projection portions have a groove portion along a rotational direction of the anvil roll, wherein, letting a direction perpendicular to the rotational direction be an intersecting direction, in a cross section of the groove portion taken along the intersecting direction, the groove portion has a pair of first side-wall portions extending from a starting edge of the groove portion to a specified position on a far side, and a pair of second side-wall portions extending from the specified position to a bottom portion of the groove portion, wherein, in the cross section, when intersection points of a line connecting starting edges of the pair of first side-wall portions in the intersecting direction and lines respectively extending the pair of second side-wall portions toward the starting edges are a first intersection point and a second intersection point, a separation distance between the starting edges of the pair of first side-wall portions is longer than a distance between the first intersection point and the second intersection point.

According to the anvil roll of Aspect 27, when manufacturing a stretchy sheet by using such an anvil roll, it becomes easier to join the first sheet and the second sheet without the elastic member protruding from the groove portion. By preventing the elastic member from being welded unintendedly, it is possible to prevent the elastic member from being cut, and by holding the elastic member securely in the groove portion, it is possible to prevent the elastic member from coming off during manufacturing.

### Aspect 28:

An anvil roll that uses for an apparatus for manufacturing a stretchy sheet in which a first sheet and a second sheet are stacked with the elastic member in between, the anvil roll including: a plurality of projection portions that project outside in a radial direction of the anvil roll, on an outer circumferential surface of the anvil roll, wherein the projection portions have a groove portion along a rotational direction of the anvil roll, wherein, letting a direction perpendicular to the rotational direction be an intersecting direction, in a cross section of the groove portion taken along the intersecting direction, a depth of the groove portion is equal to or greater than a height of the projection portion from the outer circumferential surface, and a length, in the intersecting direction, of the groove portion is shorter from the starting edge of the groove portion toward a far side.

According to the anvil roll of Aspect 28, In cases where the groove portion is shallow, there is a risk that the elastic member will protrude and be cut off during the joining step, but by using such an anvil roll, the groove portion has sufficient depth, allowing the elastic member to pass stably through the groove portion. In addition, the length in the intersecting direction (width) becomes shorter toward the far side in the groove portion, enabling the elastic member, which is placed there, more difficult to come out from the groove portion. This makes it possible to prevent the elastic member from being cut off during the joining step.

### Embodiment

### Structure of stretchy sheet 10

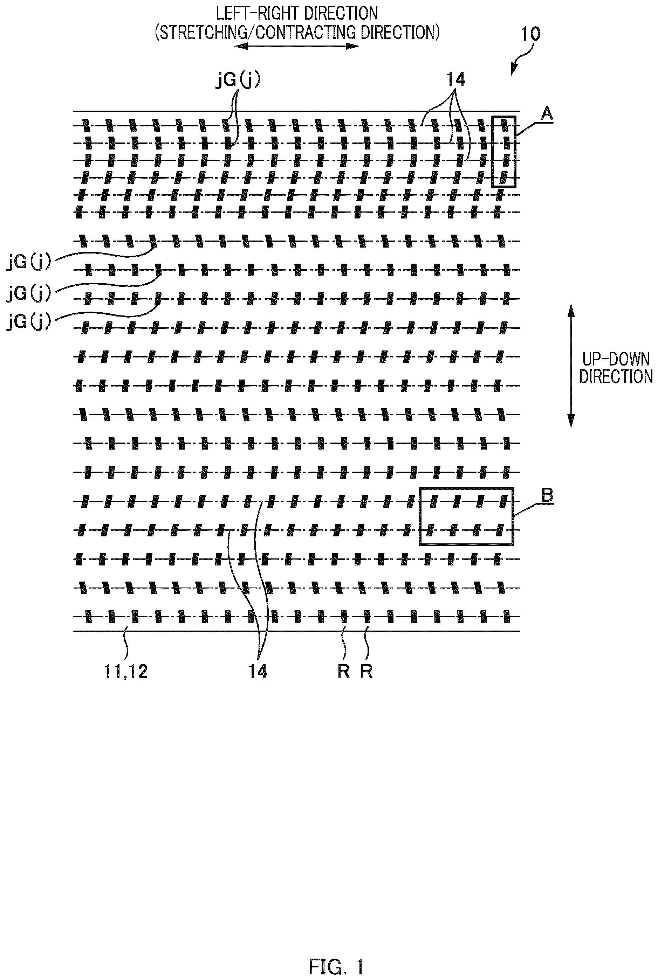

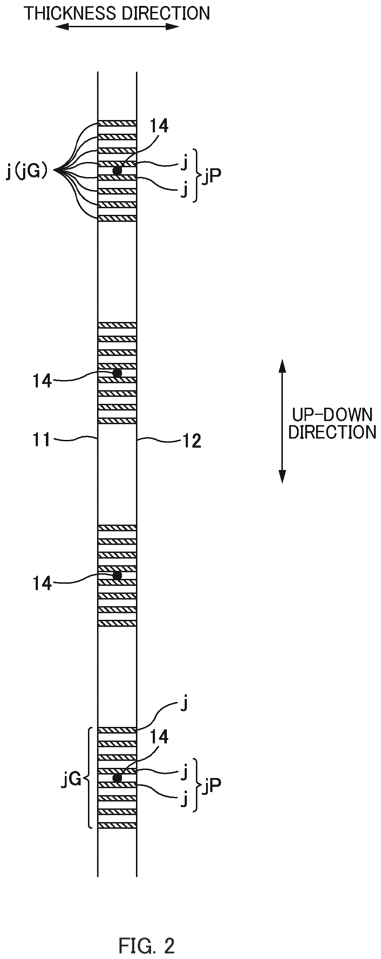

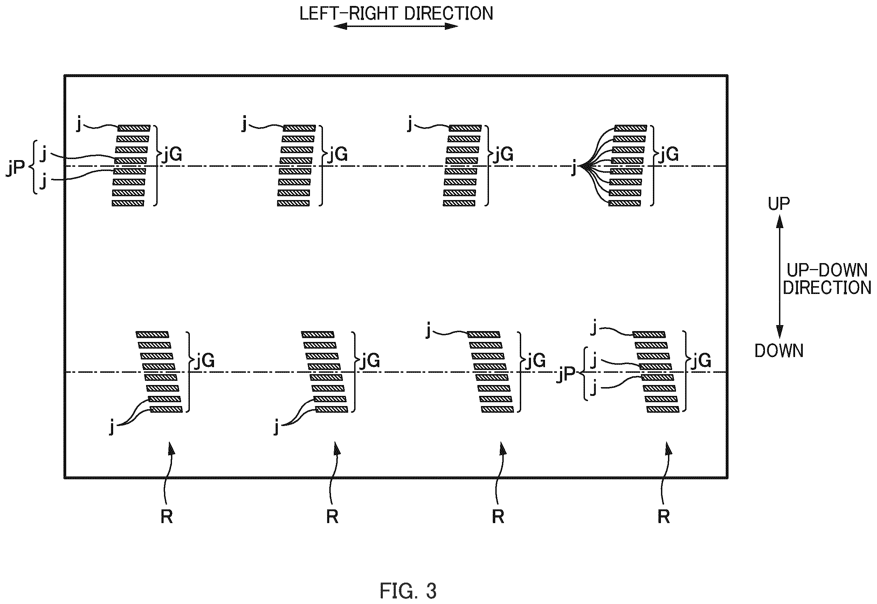

FIG. 1 is a plan view of a stretchy sheet 10 manufactured in the manufacturing method (manufacturing apparatus) of the present embodiment. FIG. 1 is a diagram of a stretchy sheet 10 in a state of being stretched to eliminate wrinkles. FIG. 2 is a schematic cross-sectional view in a portion indicated by symbol A in FIG. 1. FIG. 3 is an enlarged view of a portion indicated by symbol B in FIG. 1.

The stretchy sheet 10 has a left-right direction (stretching/contracting direction), a thickness direction, and an up-down direction that are perpendicular to one another. In addition, the stretchy sheet 10 has a first sheet 11 and a second sheet 12 that are stacked in the thickness direction, and also has a plurality of elastic members 14 (e.g., elastic strings) interposed between the first sheet 11 and the second sheet 12 and a plurality of welded portions j.

The plurality of elastic members 14 are arranged at intervals in the up-down direction, extending along the left-right direction of the stretchy sheet 10. The elastic members 14 in the stretched state are attached to the first sheet 11 and the second sheet 12. Therefore, the stretchy sheet 10 has stretchability in the direction along which the elastic members 14 extend (left-right direction). Applicable examples of the elastic members 14, are, in addition to string-like natural rubber, of various known synthetic rubbers, such as styrene rubber, urethane rubber, ester rubber, polyurethane, polyethylene, and the like.

As shown in FIGS. 1 to 3, the first sheet 11 and the second sheet 12 are intermittently joined by a plurality of the welded portions j that are discretely arranged in the up-down direction and the left-right direction. The welded portions can be formed using commonly-known welding methods such as ultrasonic welding, heat welding, or the like.

In the present embodiment, the stretchy sheet 10 has a plurality of welded-portion rows R in which a plurality of the welded portions j are arranged side-by-side in the up-down direction. The plurality of welded-portion rows R are located at intervals in the left-right direction. In addition, as shown in FIG. 1, each welded-portion row R extends in the up-down direction while meandering in the left-right direction. That is, as shown in FIG. 3, the positions, in the left-right direction, of welded portions adjacent in the up-down direction are shifted.

On the other hand, the positions, in the up-down direction, of the welded portions adjacent in the left-right direction are aligned, and therefore, as shown in FIG. 3, the positions, in the up-down direction, of the spaces which are located between the welded portions adjacent in the up-down direction and through which the elastic member 14 passes are aligned. This makes it possible for the elastic member 14 to be placed along the left-right direction. However, the present invention is not limited thereto. The positions, in the up-down direction, of the welded portions adjacent in the left-right direction may be shifted, or the elastic member 14 may pass between the welded portions whose positions in the up-down direction are different. In these cases, the stretchy sheet 10 can also have stretchability in the left-right direction.

In addition, in each welded-portion row R, the stretchy sheet 10 in the present embodiment has welded-portion groups jG in each of which a predetermined number of the welded portions j are concentrated, and the welded-portion groups jG are disposed at intervals in the up-down direction.

Welded portions j located on the respective two sides, in the up-down direction, of an elastic member 14 as shown in FIG. 3 are also referred to as a "welded-portion pair jP". The elastic member 14 being contracted in the left-right direction is sandwiched between the welded-portion pair jP, thereby restricting the position, in the left-right direction, of the elastic member 14 relative to the first sheet 11 and the second sheet 12. Details of the welded-portion pairs jP and the welded-portion groups jG will be described later. The planar shape of the welded portion j illustrated in FIG. 3 is a parallelogram, but the planar shape of the welded portion j is not particularly limited, and any shape such as a rectangle, an ellipse, or a circle can be used.

In addition, the stretchy sheet 10 in FIG. 2 includes two sheets, but for example, the following configurations are also acceptable: by folding over a single sheet, the folded-over part serves as the first sheet and the not-folded-over part serves as the second sheet; or a single sheet is folded over to form a two-layer sheet. The stretchy sheet 10 may also be made of the first and the second sheet 11, 12 and one or more additional sheets overlaid thereon. That is, the stretchy sheet 10 may have three or more layers.

### Example of use of stretchy sheet 10

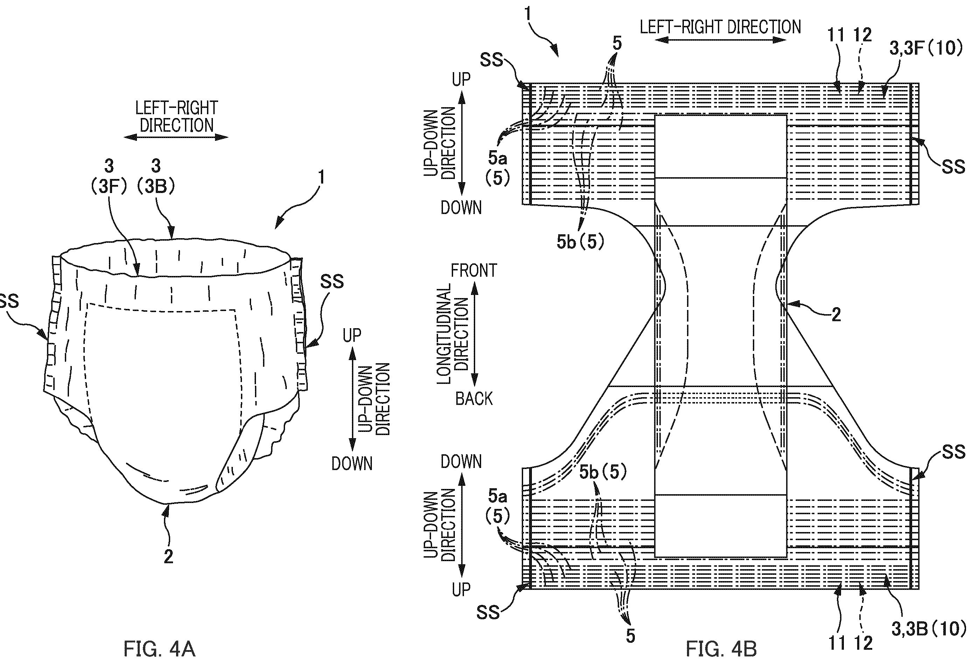

FIG. 4A is a schematic perspective view a underpants-shaped disposable diaper 1 (absorbent article) in which the stretchy sheet 10 is used. FIG. 4B is a schematic plan view of the absorbent article 1 in an unfolded state and a stretched state, viewed from the skin-side face side. The stretchy sheet 10, which is manufactured using the manufacturing method of the present embodiment (manufacturing apparatus), is used as a component of an absorbent article, for example, disposable diapers, and the like.

An underpants-shaped disposable diaper 1 (hereinafter also referred to as a "diaper") has an up-down direction and a left-right direction that intersect each other, in an underpants-shaped state when worn as shown in FIG. 4A. The diaper 1 also includes an absorbent main body 2 that absorbs and retains excrement, and a pair of waist portions 3. Of the pair of waist portions 3, the one that is attached to the ventral part of the wearer is also referred to as a front waist portion 3F, and the one that is attached to the dorsal part of the wearer is also referred to as a back waist portion 3B. The front waist portion 3F and the back waist portion 3B are joined on both sides in the left-right direction by a pair of side joining portions SS.

As shown in the unfolded state in FIG. 4B, the central portion, in the left-right direction, of the front waist portion 3F is placed on an end portion, on the one side in the longitudinal direction, of the absorbent main body 2. And the central portion, in the left-right direction, of the back waist portion 3B is placed on an end portion, on the other side in the longitudinal direction, of the absorbent main body 2. The diaper 1 in the unfolded state is folded one time at a substantial center in the longitudinal direction, and both side portions, in the left-right direction, of the front waist portion 3F and both side portions, in the left-right direction, of the back waist portion 3B are joined by welding or the like to form the pair of side joining portions SS, thereby making the diaper 1 into an underpants-shaped state.

In the front waist portion 3F and the back waist portion 3B, a plurality of elastic members 5 that stretch in the left-right direction of the diaper 1 are arranged side-by-side at intervals in the up-down direction. Thus, the front waist portion 3F and the back waist portion 3B are capable of stretching/contracting in the left-right direction of the diaper 1 and fit around the waist of the wearer. The stretchy sheet 10 can be used for the front waist portion 3F and the back waist portion 3B. In addition, the stretching/contracting direction (left-right direction) of the stretchy sheet 10 corresponds to the left-right direction of the diaper 1, and the up-down direction of the stretchy sheet 10 corresponds to the up-down direction of the diaper 1.

In cases where the stretchy sheet 10 is used as a component of the absorbent article, examples of the first sheet 11 and the second sheet 12 constituting the stretchy sheet 10 include soft sheet materials, for example, nonwoven fabrics such as spunbond nonwoven fabric and SMS (spunbond/meltblown/spunbond) nonwoven fabrics. In addition, at least either one of the first sheet 11 and the second sheet 12 may be a stretchy sheet (stretchable film or stretchable nonwoven fabric) that is capable of stretching/contracting in the stretching/contracting direction of the stretchy sheet 10. Further, stretchable film or stretchable nonwoven fabric may be sandwiched between the first sheet 11 and the second sheet 12.

Furthermore, the stretchy sheet 10 is not limited to being used for the front waist portion 3F and the back waist portion 3B. For example, the stretchy sheet 10 can be used for leg gather portions, which are placed on both side portions of the absorbent main body in the left-right direction of a diaper so that the disposable diaper fits around the wearer's legs. Since the leg gather portions stretch and contract in the longitudinal direction of the absorbent main body, the longitudinal direction of the absorbent main body corresponds to the stretching/contracting direction of the stretchy sheet 10.

In addition, in a tape-type disposable diaper (not shown), fastening tapes (hook members) extend outward from the back waist portion on both sides in the left-right direction. The stretchy sheet 10 can be used for the side panel that attaches the fastening tape to the back waist portion. The side panels stretch in the left-right direction of the diaper, and therefore the left-right direction of the diaper corresponds to the stretching/contracting direction of the stretchy sheet 10.

In addition, the stretchy sheet 10 is not limited to an underpants-shaped disposable diaper and a tape-type disposable diaper, but can also be used as a component of an absorbent article, such as a pad-shaped disposable diaper, a sanitary napkin, an underpans-shaped sanitary napkin, and the like. Further, the stretchy sheet 10 can be used not only for an absorbent article, but also for masks, cleaning sheets, and the like.

### Manufacturing apparatus and manufacturing method for stretchy sheet

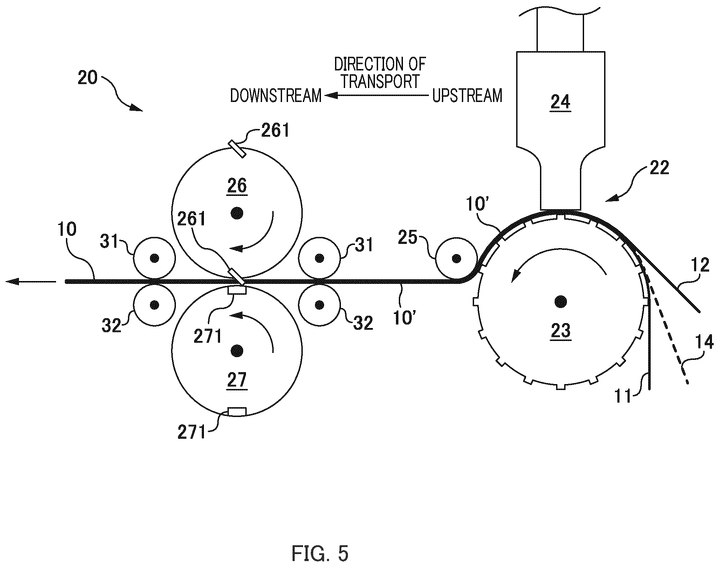

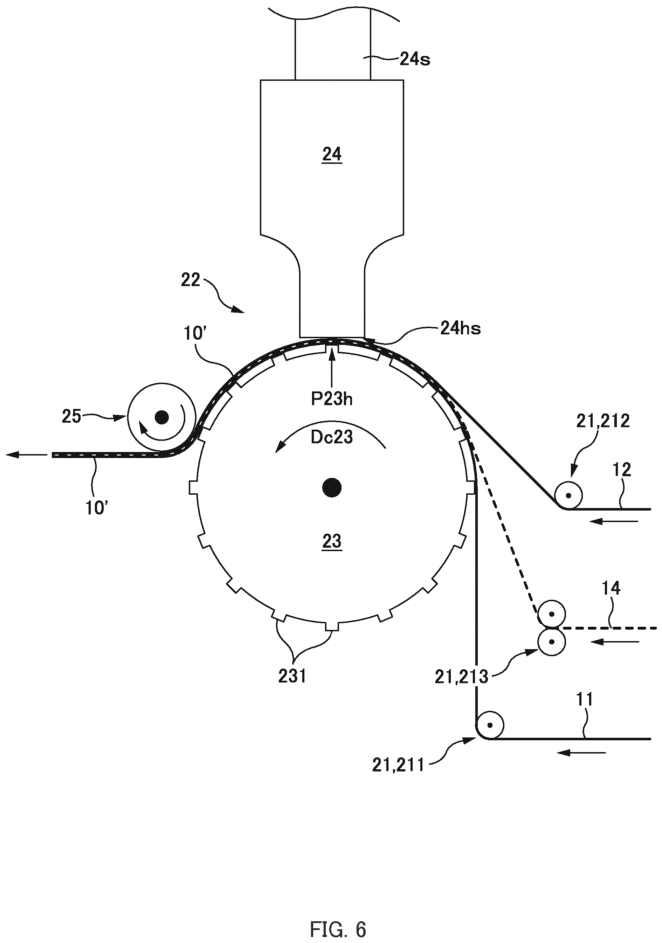

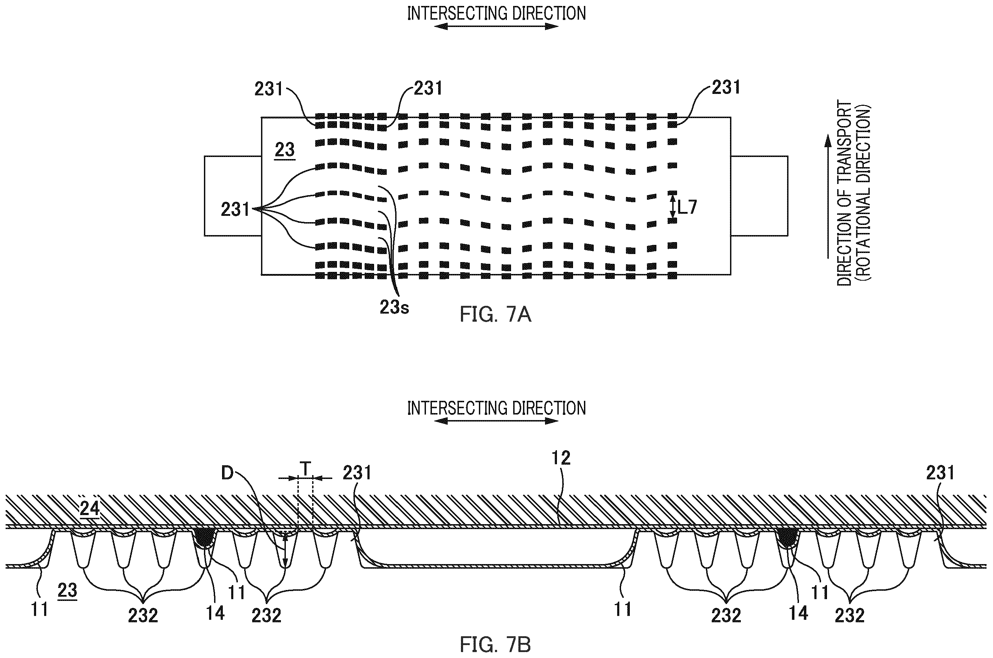

Configuration of manufacturing apparatus 20 for stretchy sheet FIG. 5 is a schematic cross-sectional view of a manufacturing apparatus 20 for a stretchy sheet in the present embodiment. FIG. 6 is a schematic cross-sectional view of an ultrasonic welding unit 22. FIG. 7A is a diagram illustrating the surface of an anvil roll 23. FIG. 7B is a schematic enlarged view of the nearest neighbors of the anvil roll 23 and a horn 24.

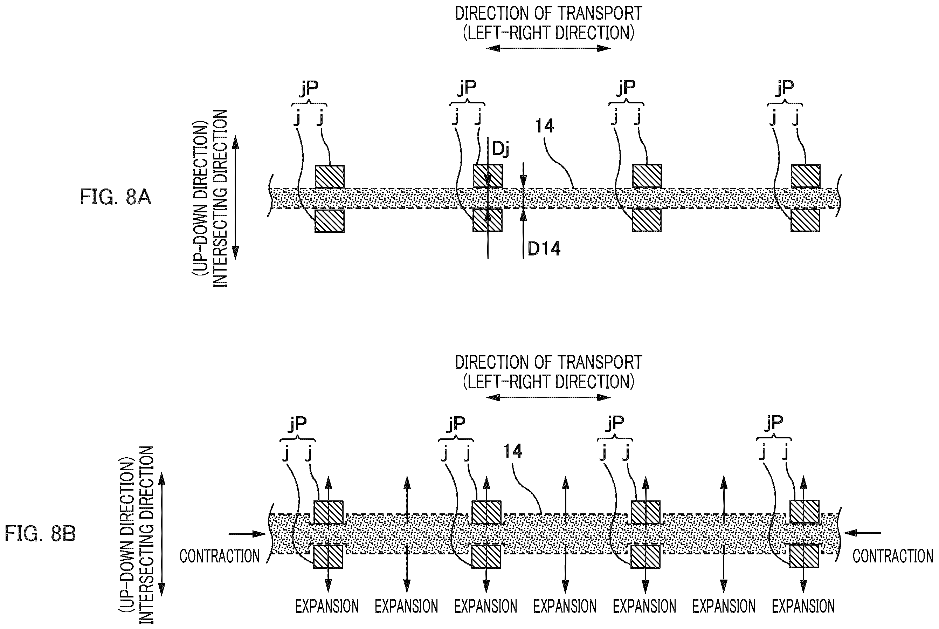

FIGS. 8A and 8B are diagrams illustrating a method for attaching the elastic member 14 by the welded portions j.

The manufacturing apparatus 20 for a stretchy sheet has a transport roller group 21 (FIG. 6), a ultrasonic welding unit 22, a discharging roll 25, a cutter roll 26, a receiving roll 27, and nip rolls 31 and 32. The stretchy sheet 10 is manufactured as a continuous sheet that continues in the left-right direction. In the manufacturing apparatus 20 for a stretchy sheet, the direction in which constituent materials continue is a direction of transport, and the direction perpendicular to the direction of transport (i.e. the up-down direction of the stretchy sheet 10) is an intersecting direction (also referred to as a CD direction) .

As shown in FIG. 6, the transport roller group 21 (supplying section) is located upstream in the direction of transport with respect to the ultrasonic welding unit 22, and transports the constituent materials of the stretchy sheet 10, to supply them the ultrasonic welding unit 22. Specifically, the transport roller group 21 includes: a first transport roller 211 which transports the continuous body of the first sheet 11; a second transport roller 212 which transports the continuous body of the second sheet 12; and a third transport roller 213 which transports multiple continuous bodies of the elastic members 14.

The first to third transport rollers 211 to 213 are rotated around a rotation axis along the intersecting direction (CD direction) by a driving source (not shown; e.g., a motor). Each of the first to third transport rollers 211 to 213 may be a pair of rolls or a single roll. Constituent materials may also be supplied to the ultrasonic welding unit 22 via a conveyor belt or the like.

Hereinafter, the continuous body of the first sheet 11, the continuous body of the second sheet 12, the continuous body of the elastic member 14, and the continuous body of the stretchy sheet 10 are also referred to simply as the first sheet 11, the second sheet 12, the elastic member 14, and the stretchy sheet 10.

The ultrasonic welding unit 22 (welded-portion forming section) is for forming the welded portions j of the stretchy sheet 10, and includes the anvil roll 23 that rotates along the direction of transport and the horn 24 that is placed at a specified position P23h in the rotational direction Dc23 of the anvil roll 23.

The horn 24 is supported by an appropriate supporting member 24s so as to be substantially immovable at the specified position P23h. The horn 24 also has a flat, vibrating surface 24hs that is located opposite the outer circumferential surface 23s of the anvil roll 23. The vibrating surface 24hs vibrates in a direction in which a gap between the surface 24hs and the outer circumferential surface 23s expands and shrinks. The frequency of the vibration is a specified value, for example, between 20 kHz and 35 kHz, and the amplitude is a specified value, for example, between 1 micron and 30 microns. Therefore, the vibrating surface 24hs ultrasonically vibrates, thereby ultrasonically welding both sheets 11 and 12 passing between the vibrating surface 24hs and the outer circumferential surface 23s. That is, the above-mentioned welded portions are formed on both sheets 11 and 12. Such vibration is generated by inputting an electrical signal of the above frequency to piezoelectric elements of a converter (not shown) connected to the horn 24.

The anvil roll 23 is supported rotatably around a rotation axis along the CD direction, by an appropriate supporting member (not shown), such as a bearing. The roll 23 is driven to rotate by a driving force from a servo motor (not shown) of the driving source. In addition, the roll 23 is wound by the following three materials, with almost no relative slippage between the roll 23 and the outer circumferential surface 23s: the first sheet 11 transported from the above-mentioned first transport roller 211; the second sheet 12 transported from the above-mentioned second transport roller 212; and the elastic members 14 transported from the above-mentioned third transport roller 213. Therefore, by making the anvil roll be driven to rotate, the first sheet 11, the second sheet 12, and the elastic members 14 are transported along the outer circumferential surface 23s of the anvil roll 23, at the same transport speed value as the circumferential speed value of the anvil roll 23. That is, both of the continuous sheets 11 and 12 and the elastic members 14 are transported along transport paths curving along the outer circumferential surface 23s.

Here, the circumferential speed value (mpm) of the first transport roller 211 of the first sheet 11 and the circumferential speed value (mpm) of the second transport roller 212 of the second sheet 12 are each substantially the same as the circumferential speed value (mpm) of the anvil roll 23. Therefore, the first sheet 11 and the second sheet 12 are wound around the anvil roll 23 substantially without stretching, but are taut enough not to slacken. On the other hand, the circumferential speed value (mpm) of the transport roller 213 of the elastic members 14 is a value obtained by multiply the circumferential speed value (mpm) of the anvil roll 23 and an inverse of an approximately stretch factor. Therefore, regarding the elastic members 14, while passing between the third transport roller 213 and the anvil roll 23, they stretch to the above stretch factor and wind, being in the stretched state, around the anvil roll 23.

In this example, the order of winding around the anvil roll 23 is as follows: the first sheet 11 winds around first, then the elastic members 14 wind around, and finally the second sheet 12 winds around. As a result, these three materials are in a state where the elastic members 14 are interposed between the first sheet 11 and the second sheet 12 on the outer circumferential surface 23s of the anvil roll 23.

Furthermore, the outer circumferential surface 23s of the anvil roll 23 has a plurality of projection portions 231 that project outside in the radial direction of the anvil roll 23, as shown in FIG. 6. The projection portions 231, together with the horn 24, ultrasonically welds constituent materials, to form the welded portions j. Therefore, the projection portions 231 are placed in accordance with the placement pattern of the welded portions j in the stretchy sheet 10.

In addition, the plurality of projection portions 231 provided in the outer circumferential surface 23s of the anvil roll 23 has a plurality of groove portions 232 along the rotational direction of the anvil roll 23. In the plurality of groove portions 232, the elastic members 14 are placed when forming the above-mentioned the welded portions j, and the details thereof will be described later.

The discharging roll 25 is a roll that discharges the semifinished products of the stretchy sheet, where the welded portions j have been formed, to the cutting process of the elastic members 14. The discharging roll 25 is rotated around a rotation axis along the CD direction by a driving source (not shown).

The cutter roll 26 and the receiving roll 27 (cutting section) are oppositely positioned rolls on the downstream side in the direction of transport with respect to the ultrasonic welding unit 22. The cutter roll 26 and the receiving roll 27 are rotated around a rotation axis along the CD direction by the driving source (not shown).

The outer circumferential surface of the cutter roll 26 has a cutter blade 261 protruding outside in the radial direction of the cutter roll 26. In FIG. 5, two cutter blades 261 are provided with spaces in the rotational direction of the cutter roll 26, but the number of cutter blades is not limited to this. The spacing of the cutter blades 261 in the rotational direction corresponds to the spacings of the cutting positions of the elastic members 14. Further, the plurality of elastic members 14 that are located side-by-side in the CD direction (up-down direction) in the stretchy sheet 10 may be cut at a cutter blade 261 extending in the CD direction, or may be cut at a plurality of cutter blades 261 that are provided for each of the elastic members 14 and that are located side-by-side in the CD direction.

The outer circumferential surface of the receiving roll 27 has receiving blades 271 each of which receive the cutter blade 261 of the cutter roll 26. However, the receiving roll 27 does not have to the receiving blade 271, but may be a roll that has, for example, a smooth outer circumferential surface, or may be a roll that has a groove portion receiving the cutter blade 261.

The pair of nip rolls 31, 32 are intended to pinch elastic members 14 and restrict the contraction movement of the elastic members 14 when the cutter roll 26 and the receiving roll 27 are cutting the elastic members 14. In the manufacturing apparatus 20 of the stretchy sheet shown in FIG. 5, the pairs of nip rolls 31, 32 are each provided on the downstream side and the upstream side of the cutter roll 26 and the receiving roll 27, but this is not limited to this. A manufacturing apparatus may be acceptable in which a pair of nip rolls 31, 32 is provided on either one side of the downstream side and the upstream side.

### Method for manufacturing stretchy sheet 10: Step of supplying to anvil roll

In a method for manufacturing the stretchy sheet 10 using the manufacturing apparatus 20 configured as above, first, the transport roller group 21 (supplying section) supplies the first and second sheets 11, 12 and the elastic members 14 to the anvil roll 23, as shown in FIG. 6. Specifically, the elastic members 14 are supplied so as to be located outside the first sheet 11 and inside the second sheet in the radial direction of the anvil roll 23. Thus, the elastic members 14 in a state of being stretched in the direction of transport (left-right direction) are placed between the first sheet 11 and the second sheet 12.

### Method for manufacturing stretchy sheet 10: joining step

Next, the first and second sheets 11, 12 and the elastic members 14 wound around the anvil roll 23 are transported to the ultrasonic welding unit 22 by the rotation of the anvil roll 23 and pass between the anvil roll 23 and the horn 24. At this time, the ultrasonic welding unit 22 (welded-portion forming section) forms the plurality of welded portions j by joining the first sheet 11 and the second sheet 12 together by ultrasonic welding (pressure bonding).

Specifically, the first and second sheets 11, 12 are melted and joined by receiving ultrasonic vibrations from the horn 24 at positions corresponding to the projection portions 231 of the anvil roll 23. At this time, the elastic members 14 are located at the groove portions 232 of the anvil roll 23 (see FIGS. 7A and 7B) and are not ultrasonically welded. In FIG. 6 and other figures, for ease of understanding, the elastic members 14 are located above the projection portions 231 of the anvil roll 23. However, in reality, the elastic members 14 are located in the groove portions 232.

As described above, the plurality of welded portions j also serve as a role of restricting the positions, in the left-right direction, of the elastic members 14 relative to the first sheet 11 and the second sheet 12, that is, a role of attaching the elastic members 14 to the first and second sheets 11, 12. For this purpose, in the present embodiment, as shown in FIG. 1, a plurality of the welded-portion pairs jP located on both sides of each of the elastic members 14 in the up-down direction of the stretchy sheet 10 are formed at intervals in the left-right direction of the stretchy sheet 10.

Furthermore, the welded-portion forming section forming the welded portions j is not limited to the ultrasonic welding unit 22, but may be any device capable of joining a plurality of sheets together by pressure bonding. For example, the heat sealing device may be used to form the welded portions j. An example of a heat sealing device is a pair of heated rollers, where one of the rollers has projection portions corresponding to the welded portions j on the outer circumferential surface, and the other one of the rollers is a roller that receives the above projection portions by a smooth outer circumferential surface.

Also, while FIG. 6 illustrates an example of the ultrasonic welding unit 22 using the non-rotating horn 24, the present invention is not limited to this. For example, the ultrasonic welding unit 22 may be used which is constituted by a pair of rollers (a horn roll and an anvil roll). Further, the following configuration is acceptable: an ultrasonically-vibrating horn is a roller-shaped device; and an anvil is a non-rotating device having a flat surface that receives ultrasonic vibrations. In addition, the vibrating surface on the horn side may be made an uneven surface, and the surface on the anvil side may be made a smooth surface.

### Method for manufacturing stretchy sheet 10: Step of cutting elastic members 14

The manufacturing method of the stretchy sheet 10 in the present embodiment includes a step of cutting the elastic members 14 after joining the first sheet 11 and the second sheet 12 by forming the welded portions j. Therefore, as shown in FIG. 5, the stacked body 10' of the first and second sheets 11, 12 in which the welded portions j, and of the elastic members 14 are formed is transported by the discharging roll 25 and passes between the cutter roll 26 and the receiving roll 27 (cutting section). At this time, the cutter blade 261 is inserted into the stacked body 10' supported by the receiving blade 271, and the elastic members 14 are cut.

Note that, in the step of cutting the elastic members 14, the first sheet 11 and the second sheet 12 may also be cut and separated together with the elastic members 14, or only the elastic members 14 may be cut and separated without separating the first sheet 11 and the second sheet 12.

For example, when cutting the continuous body of the stretchy sheet 10 into the product size of the diaper 1 (absorbent article) shown in FIG. 4A, it is recommended to cut and separate the first sheet 11 and the second sheet 12 together with the elastic members 14. That is, the absorbent main body 2 is placed on the stretchy sheet 10 which includes the first sheet 11 and the second sheet 12, and then they is folded one time in the center, in the longitudinal direction, of the diaper 1, and then the pair of side joining portions SS are formed on both sides, in the left-right direction, of the front waist portion 3F and the back waist portion 3B, and then the first sheet 11 and the second sheet 12 are cut and separated together with the elastic members 14.

On the other hand, there are cases where the stretchability is partially removed from the stretchy sheet 10. For example, in cases where the stretchy sheet 10 is used for the front waist portion 3F and the back waist portion 3B, as in the diaper 1, there are cases where the stretchability is removed from the areas where the front waist portion 3F and the back waist portion 3B overlap the absorbent main body 2, in order to ensure the flatness of the absorbent main body 2, and the like. In this case, it is recommended to cut and separate the central portions of the elastic members 14, which extend in the left-right direction in the stretchy sheet 10, so that the first sheet 11 and the second sheet 12 do not separate. However, in cases of separating the first sheet 11 and the second sheet 12 together with the elastic member 14, no cutting marks will remain on the stretchy sheet 10, making it possible to improve the appearance of the stretchy sheet 10.

### Positional restriction of elastic members 14

Then, after the cutting step by the cutting section is performed, the elastic members 14 that have contracted are sandwiched between the welded-portion pairs P, and the positions, in the direction of transport (left-right direction), of the elastic members 14 are restricted. Specifically, as shown in FIG. 8A, in the intersecting direction (up-down direction, CD direction), the distance Dj of the welded-portion pair jP located on both sides of an elastic member 14 is greater than or equal to the diameter D14, in the intersecting direction, of the elastic member 14 that is stretching at the time of forming the welded portions j. Furthermore, the distance Dj is set to be smaller than the diameter, in the intersecting direction, of the elastic member 14 in an unloaded state where it has a natural length. The elastic member 14 in the stretched state is thinner than the elastic member 14 in the natural state by the amount of elongation. Therefore, after the continuous bodies of the elastic members 14 are cut subsequent to the formation of the welded portions j, the stretched state of the elastic member 14 is relaxed, and as shown in FIG. 8B, the elastic member 14, which is contracting in the direction of transport while expanding in the intersecting direction, is sandwiched between the welded-portion pairs jP. Therefore, the positions, in the left-right direction, of the elastic members 14 are restricted, thereby making the elastic members 14 attached to the sheets.

In this way, the stretchy sheet 10 is manufactured in which the first sheet 11 and the second sheet are stacked with the elastic members 14 interposed therebetween. However, in cases where the width between the welded portions decreases in order to ensure that the elastic members 14 securely sandwiched, the elastic members 14 may be torn. On the other hand, in cases where the width between the welded portions between which the elastic members 14 are sandwiched increases in order not to cut them, there is a risk that the elastic members 14 will come off during manufacturing. That is, it has been difficult that the plurality of elastic members 14 arranged in the manufacturing process of the stretchy sheet 10 keep attached without cutting them.

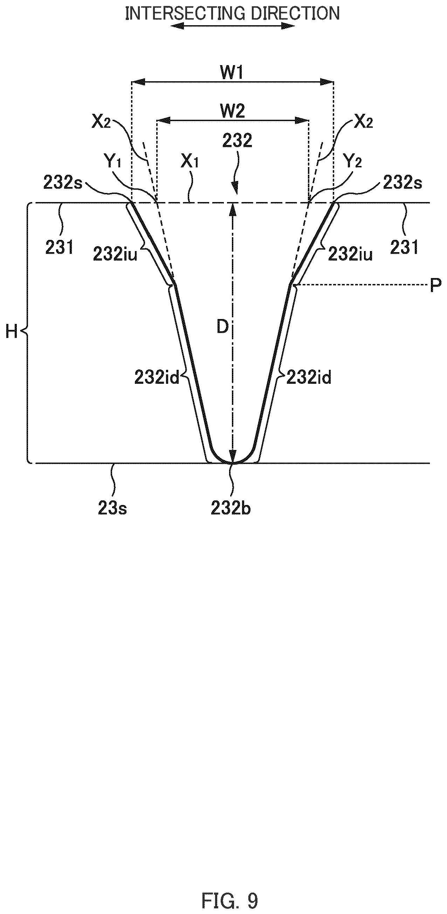

In this regard, in the present embodiment, in order to stably place the elastic members 14 to the groove portions 232 provided in the projection portions 231 of the anvil roll 23, each groove portion 232 has the following configuration. FIG. 9 is a cross-sectional view showing a cross section of the groove portion 232 taken along the intersecting direction. As shown in FIG. 9, in the cross section, the groove portion 232 in the present embodiment has a pair of first side-wall portions 232iu extending from the starting edge 232s of the groove portion 232 to a specified position P on the far side, and a pair of second side-wall portions 232id extending from the specified position P to the bottom portion 232b of the groove portion 232. In this cross section, when intersection points of a line X1 connecting the starting edges 232s, 232s of the pair of first side-wall portions 232iu in the intersecting direction and lines X2 respectively extending the pair of second side-wall portions 232id toward the starting edges 232s are the first intersection point Y1 and the second intersection point, the separation distance W1 between the starting edges 232s, 232s of the pair of first side-wall portions 232iu is longer than the distance W2 between the first intersection point Y1 and the second intersection point Y2 (W1 > W2).

Here, the separation distance W1 between the starting edges 232s, 232s of the first side-wall portions 232iu defines the opening width of the groove portion 232. In the present embodiment, the groove portion 232 is configured such that a groove width (length in the intersecting direction) is wider toward the opening from a specified position P located on the far side with respect to the opening. With this configuration, the elastic members 14 become easier to enter the groove portions 232, and when forming the welded portions j in the above-mentioned joining step, the first sheet 11 and the second sheet 12 become easier to join without the elastic members 14 protruding from the groove portions 232. By preventing the elastic members 14 from being welded unintendedly in the joining step, it is possible to prevent the elastic members 14 from being cut, and by carrying out the step of supplying the elastic members 14 while steadily passing them through the groove portions 232 and the joining step, it is possible to prevent the elastic members 14 from coming off during the steps. Note that the separation distance (opening width) W1 is preferably between 0.2 mm and 0.4 mm.

Additionally, it is preferable that the depth D of the groove portion 232 is equal to or greater than the height H of the projection portion 231 from the outer circumferential surface 23s of the anvil roll 23 (D ≥ H). As shown in FIG. 9, in the present embodiment, the depth D of the groove portion 232 and the height H of the projection portion 231 are approximately the same, but the depth D of the groove portion 232 may be greater than the height H of the projection portion 231 (in that case, the groove portion is formed further inward in the radial direction than the outer circumferential surface 23s). In addition, the length (width), in the intersecting direction, of the groove portion 232 becomes shorter from the starting edges 232s of the groove portion 232 toward the far side. That is, since the shape narrows toward the far side, the elastic members 14 easily enter the groove portions 232 but are less likely to come out. In cases where the depth of the groove portions 232 is shallow, the elastic members 14 will come out when placed, and there will be a risk of the elastic members 14 being cut off during the joining step. However, as in cases of the groove portions 232 of the present embodiment, by having a sufficient depth, the elastic members 14 can pass stably through the groove portions 232, making it less likely for the elastic members 14 to be cut off or damaged during the joining step. The depth D of the groove portions 232 is preferably 0.3 mm to 0.5 mm.

In the present embodiment, as shown in FIG. 9, the groove portion 232 has a two-stage width (length) in the intersecting direction, namely a part consisting of the first side-wall portions 232iu and a part consisting of the second side-wall portions 232id, but the present invention is not limited to this. As long as the depth D of the groove portion 232 is greater than or equal to the height H of the projection portion 231, a configuration is acceptable in which, for example, the groove portion 232 does not have the first side-wall portions 232iu and the second side-wall portions 232id, and have a shape that tapers steadily toward the far side at a specified inclination angle (a so-called approximate V-shape).

Also, as shown in FIG. 7B, in the supplying step and the joining step, one sheet of the first sheet 11 and the second sheet 12 (here, the first sheet 11) also enters the groove portions 232 together with the elastic members 14, so that the one of the sheets may also be subjected to a pulling force during transport. Therefore, in cases where the groove portion 232 is shallow, the one of the sheets (first sheet 11) will rise and become difficult to enter inside. Therefore, the deeper the groove portions 232 are, the easier it is for the first sheet 11, along with the elastic members 14, to enter the groove portions 232. Furthermore, in cases where the height H of the projection portions 231 from the outer circumferential surface 23s is too high, it will be difficult for the first sheet 11 to enter the groove portions 232 because the first sheet 11 being pulled which is trying to follow the outer circumferential surface 23s (and the projection portion 231). Therefore, it is easier for the first sheet 11 to enter the groove portions 232 in cases where the height (H) of the projection portions from the outer circumferential surface 23s is low and the depth (D) of the groove portions 232 is deep.

In addition, in the present embodiment, the groove portions 232 not only has sufficient depth (D), but also the opening width (corresponding to W1) of the groove portions 232 is wide, making it easier for the elastic members 14 to be placed without protruding from the groove portion 232. Therefore, the elastic members 14 are more likely to tightly stay within the groove portions 232 and the elastic members 14 is less susceptible to the influence of the projection portions 231.

Also, as shown in FIG. 7B, the projection portions 231 of the anvil roll 23 have a plurality of the groove portions 232 for each elastic member 14, which are located spaced apart in the intersecting direction, and in the present embodiment, seven groove portions 232. Each projection portion 232 having seven groove portions form the welded-portion group jG (FIG. 3) of the stretchy sheet 10 mentioned above. In addition, the projection portions 231 each having a plurality of the groove portions 232 are provided at a specified interval in the intersecting direction, and are arranged along the intersecting direction (CD direction) while meandering in the rotational direction of the anvil roll 23. In the projection portion 231, since multiple groove portions 232 are provided for a single elastic member 14, even if the placement of the elastic member 14 is slightly shifted during the manufacturing of the stretchy sheet 10 (supplying step), as long as the elastic member 14 is placed at any of the seven groove portions 232 mentioned above, the joining step can be carried out without any cutting or coming off. It should be noted that the number of the groove portions 232 for a single elastic member 14 is not limited to the above-mentioned number.

Also, as shown in FIG. 7B, in a cross section of the groove portions 232 along the intersecting direction, the depth D of the groove portions 232 is greater than the spacing T of the groove portions 232 in the intersecting direction. In other words, since the depth D of the groove portions 232 is large, the elastic members 14 can pass more stably through the groove portion 232. The depth D of the groove portions 232 is not limited to being longer than the spacing T of the groove portions 232, but may be any length equal to or greater than the spacing T.

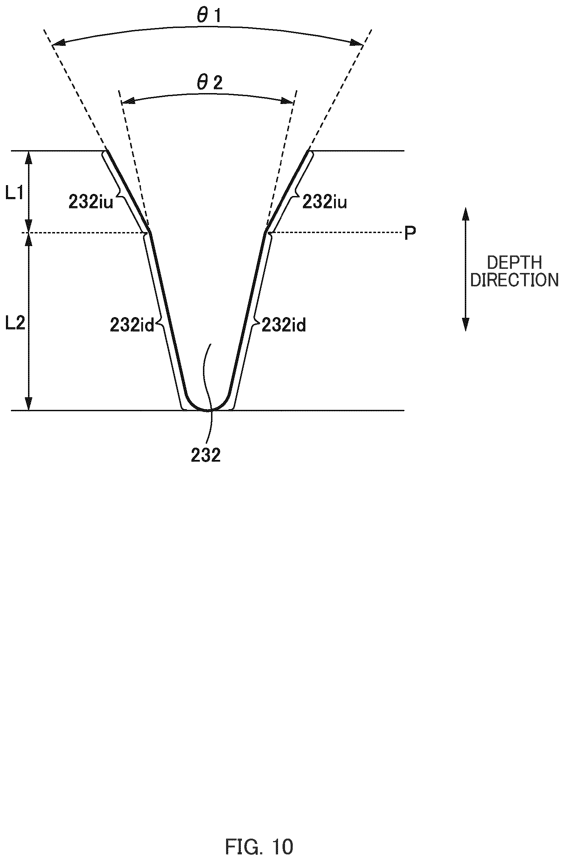

FIG. 10 is a diagram illustrating the configuration of the groove portion 232. As shown in FIG. 10, in a cross section of the groove portion 232 taken along the intersecting direction, it is preferable that an angle θ1 formed by the pair of first side-wall portions 232iu is equal to or greater than twice the angle θ2 formed by the pair of second side-wall portions 232id. In the present embodiment, the angle θ1 is 55°, but is not limited to this, and the angle θ1 is preferably from 45° to 135°. In the present embodiment, the angle θ2 is 25°, but is not limited to this, and the angle θ2 is preferably from 15° to 55°. Such a shape of the groove portion 232 makes it easier for the elastic member 14 to enter the groove portion 232 and less likely to come out, making it less likely for the elastic member 14 to be cut off or damaged during the supplying step and the joining step.

Also, as shown in FIG. 10, in a cross section of the groove portion 232 taken along the intersecting direction, in the depth direction of the groove portion 232, the length L2 of the portion of the groove portion 232 formed by the pair of second side-wall portions 232id is longer than the length L1 of the portion of the groove portion 232 formed by the pair of first side-wall portions 232iu (L2 > L1). Because the length L2 of the portion formed by the pair of second side-wall portions 232ids, i.e., the length, on the far side (bottom portion side), of the groove portion 232, is long, it becomes easier to accommodate the elastic member 14 up to the far side of the groove portion 232 when passing the elastic member 14 through the groove portion 232. As a result, during the joining step, the elastic member 14 is less likely to come out of the groove portion 232.

Additionally, it is preferable that, in a cross section of the groove portion 232 taken along the intersecting direction, the depth D of the groove portion 232 is longer than the outer diameter of the elastic member 14 when the elastic member 14 is stretched to its maximum stretchable length in the direction of transport. This makes it difficult for the elastic member 14 placed in the stretched state to come out of the groove portion 232 during manufacturing, and allows the joining step to be carried out without cutting the elastic member 14.

Also, it is preferable that, in a cross section of the groove portion 232 taken along the intersecting direction, the depth D of the groove portion 232 is longer than the maximum outer diameter of the elastic member 14 which has contracted after the cutting step. By making the groove portion 232 have a sufficient depth as described above, the elastic member 14 is less likely to come out of the groove portion 232, making it possible to carry out the joining step without cutting the elastic member 14.

The measurement methods for the outer diameter of the elastic member 14 when the elastic member 14 is stretched to its maximum stretchable length and for the maximum outer diameter of the elastic member 14 after contraction are as follows. First, for measuring the outer diameter of the elastic member 14 when stretching, the width of the elastic member 14 when the stretchy sheet 10 is stretched to its maximum stretchable length is measured using a digital microscope (VHX7000 manufactured by Keyence Corporation). Similar measurements are made at 10 locations, and the average value thereof is the outer diameter of the elastic member 14 when being stretched to its maximum stretchable length.

In addition, for measuring the maximum outer diameter of the elastic member 14 when contracted, the elastic member 14 is taken from the stretchy sheet 10, and the width of the elastic member 14 alone in the natural state is measured using the digital microscope described above. Similar measurements are made at 10 locations, and the average value thereof is the maximum outer diameter during contraction.

Alternatively, the measurements may be made at a location where the outer diameter of the cross section of the elastic member 14 in the stretchy sheet 10 is largest. In this case, a sample for cross-section measurement can be obtained by cutting the stretchy sheet 10 which has immersed in liquid nitrogen and solidified. Note that it may be solidified using a commercially available cooling spray. A magnified image of the sample is taken with a microscope (VHX7000 manufactured by Keyence Corporation) and measurement is performed.

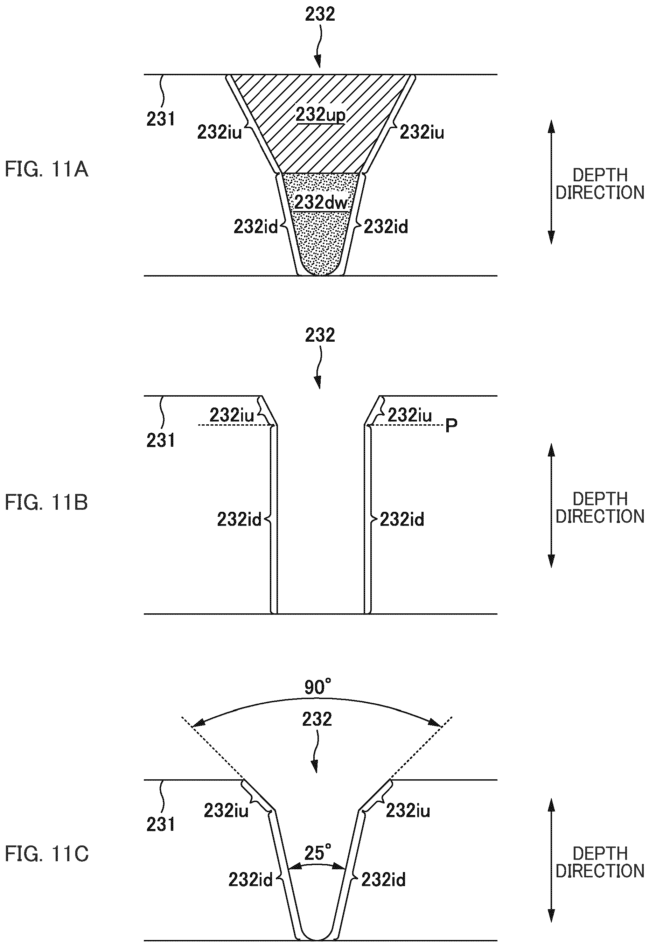

FIGS. 11A to 11C are each a diagram showing modified examples of the groove portion 232. In the groove portion 232 shown in FIG. 11A, in a cross section of the groove portion 232 taken along the intersecting direction, the area ratio occupied by a portion 232up of the groove portion 232 (hatched area in FIG. 11A) that corresponds to the pair of first side-wall portions 232iu, in the depth direction, is larger than the area ratio occupied by a portion 232dw (area indicated by fine dots in FIG. 11A) that corresponds to the pair of second side-wall portions 232id. In this way, by the groove portion 232 having a configuration in which the first side-wall portion 232iu side (starting edge 232s side) is larger, it becomes easier for the elastic member 14 to enter the groove portion 232, making it possible to place the elastic member 14 stably.

In the groove portion 232 shown in FIG. 11B, the pair of first side-wall portions 232iu are inclined so that the groove portion 232 narrows toward the far side (specified position P) of the groove portion 232, but the pair of second side-wall portions 232id extends from the specified position P toward the far side without inclining (without the length in the intersecting direction (width) reducing). That is, compared with the groove portion 232 of the present embodiment described above and the groove portion 232 shown in FIG. 11A, the groove portion 232 has a larger area on the far side. With the groove portion 232 having this type of shape, even if the elastic member 14 (elastic string etc.) is thick, it can be stably arranged within the groove portion 232, and the elastic member 14 can be prevented from coming out of the groove portion 232.

In the groove portion 232 shown in FIG. 11C, the angle between the pair of first side-wall portions 232iu is 90°, and the opening of the groove portion 232 is widely spaced. In cases where the opening is wide, it is easy for the elastic member 14 to enter, but it is also possible that it may fall out during the steps. However, the groove portion 232 shown in FIG. 11C has a deep depth at a portion of the second side-wall portions 232id and has a tapered shape toward the far side, so that the elastic member 14 that enters from the wide side of the first side-wall portions 232iu is pushed into the second side-wall portion 232id side, thereby preventing it from falling out. Such a groove portion 232 can make it easier for the elastic member 14 to enter the groove portion 232 but difficult for it to come off.

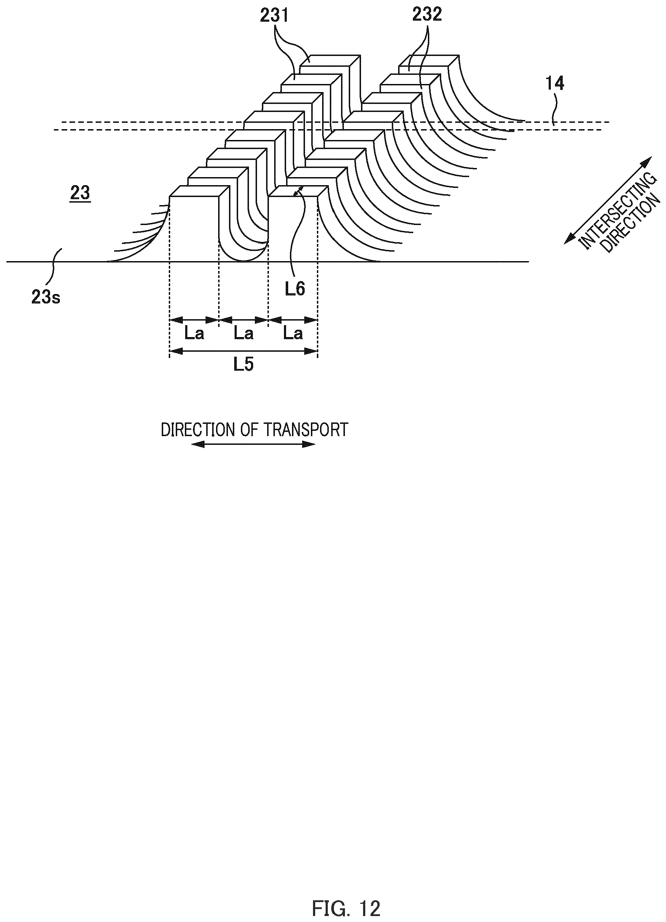

FIG. 12 is a perspective view illustrating the projection portion 231. The length L5, in the direction of transport, of the projection portion 231 corresponds to the length, in the left-right direction, of each of the welded portions j of the stretchy sheet 10, which are formed by the projection portion 231. In the present embodiment, the length L5, in the direction of transport, of the projection portion 231 is 1.5 mm, and the projection portion 231 has a shape in which the center, in the direction of transport, obtained by dividing the length L5 into three equal parts is cut out (a so-called hollow shape). Therefore, the length La shown in FIG. 12 is 0.5 mm (1.5 mm/3). Note that the length of the hollowed portion does not have to be 1/3 of the length, in the direction of transport, of the projection portion 231. Preferably, the length L5, in the direction of transport, of the projection portion 231 is in the range of 0.2 mm to 3.0 mm. By the projection portion 231 having the hollow shape described above, it is possible to prevent the welded portion j from becoming too hard.

In the present embodiment, the length L6, in the intersecting direction, of the projection portion 231 that forms the welded portions j (FIG. 12) is 0.25 mm. In addition, the length L6, in the intersecting direction, is preferably in the range of 0.2 mm to 0.5 mm. Also, it is preferable that the length L5, in the direction of transport, of the projection portion 231 is longer than the length L6, in the intersecting direction, of the projection portion 231 (L5 > L6). The elastic member 14 in a stretched state is placed along the direction of transport to the groove portion 232 (the elastic member 14 is shown by a virtual line (dotted line) in FIG. 12), and therefore, by making the length L5, in the direction of transport, of the projection portion 231 longer, this makes it difficult for the elastic member 14 to come off while reliably welding the first sheet 11 and the second sheet 12 together.

Returning to FIG. 7A, it is preferable that the distance L7 between projection portions 231 adjacent in the direction of transport (i.e., equivalent to the distance between the welded-portion rows R adjacent in the left-right direction of the stretchy sheet 10) is longer than the length L5, in the direction of transport, of the projection portion 231 described above (L7 > L5). In cases where the distance L7 between adjacent the projection portions 231 becomes too short, the distance in the left-right direction between welded-portion rows R formed by them will become too short, causing a risk of stiffness occurring due to multiple welded portions j. However, by making the distance L7 longer than the length L5, in the direction of transport, of the projection portion 231, the occurrence of stiffness is reduced.

Next, the elastic members 14 that are supply in the above-mentioned supplying step will be specifically described. In the supplying step, the elastic member 14 is supplied to the anvil roll 23 together with the first sheet 11 and the second sheet 12, more specifically, a plurality of elastic members 14 in a state of stretching in the direction of transport are supplied to the anvil roll 23 with the elastic members 14 placed spaced apart in the intersecting direction between the first sheet 11 and the second sheet 12. The plurality of elastic members 14 in the stretched state that are supplied to the anvil roll 23 in the supplying step may include a first elastic member 14a and a second elastic member 14b having different thicknesses.

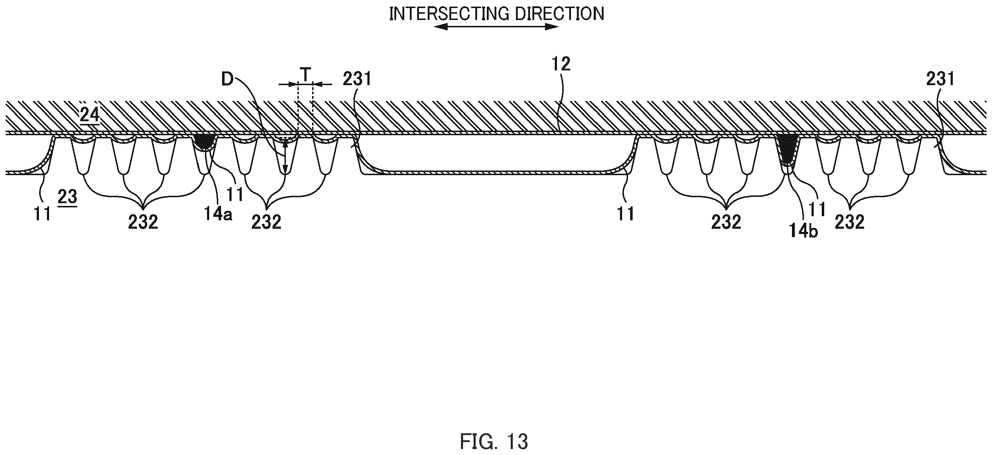

FIG. 13 is a diagram illustrating the joining step in a state in which the first elastic member 14a and the second elastic member 14b are interposed. Here, it is assumed that the first elastic member 14a is an elastic member which is thinner in the stretched state than the second elastic member 14b. As shown in FIG. 13, in the joining step of the present embodiment, the first elastic member 14a and the second elastic member 14b are both accommodated within the groove portions 232, and in this state, the joining step is carried out. In particular, even the second elastic member 14b, which has a large thickness in the stretched state, can be stably placed by being pushed into the far side of the groove portion 232 since the groove portion 232 in the present embodiment has a large depth.

In this way, by reliably placing the first elastic member 14a and the second elastic member 14b in the groove portion 232, the welded-portion pairs jP can be formed, in the joining step, for each of the first elastic member 14a and the second elastic member 14b so that the damaged portion where a part of the surface has damaged is not formed. Furthermore, by forming the welded-portion pair jP in this manner, the first elastic member 14a and the second elastic member 14b that have contracted after the cutting step are sandwiched between the corresponding welded-portion pair jP, thereby restricting the positions, in the direction of transport, of the first elastic member 14a and the second elastic member 14b. In other words, in cases where the elastic members 14 are, for example, elastic strings, even if the first elastic member 14a and the second elastic member 14b are elastic strings having different yarn counts and having different stretch factors, the joining step can be performed without the damaged portion occurring on the surface of both, and it is possible to restrict their positions in the direction of transport without the first elastic member 14a and the second elastic member 14b coming off.

As shown in FIG. 13, in the joining step of the present embodiment, the welded-portion pair jP can be formed without the first elastic member 14a and the second elastic member 14b coming out of from the groove portions 232 in which they are respectively placed. Even with the first elastic member 14a and the second elastic member 14b that have different thicknesses in the stretched state, the welded-portion pair jP is formed in a state where the elastic members do not come out of the groove portions 232 (when joining, they do not come out of the groove portions 232 to be caught by the projection portions 231). Therefore, both the first elastic member 14a and the second elastic member 14b are fixed in place without the welded-portion pair jP being placed thereon (cutting them).

Also, as shown in FIG. 13, it is preferable that, in a cross section of the groove portion 232 taken along the intersecting direction, the depth D of the groove portions 232 is equal to or greater than the spacing T of the groove portions 232 in the intersecting direction. Since the depth of the groove portions 232 is large, even with the elastic members 14 having different thicknesses in the stretched state (first elastic member 14a and second elastic member 14b), the elastic members 14 can pass through the groove portions 232 more stably.

Also, it is preferable that, in a cross section of the groove portion 232 taken along the intersecting direction, the depth D of the groove portion 232 is longer than the outer diameter of each of the first elastic member 14a and the second elastic member 14b when each of the first elastic member 14a and the second elastic member 14b is stretched to its maximum stretchable length in the direction of transport. With this configuration, even if there is variation in stretched state between the first elastic member 14a and the second elastic member 14b, which have different thicknesses, it is possible to carry out the step of passing the elastic members 14a and 14b stably through the groove portion 232.

Also, it is preferred that, in a cross section of the groove portion 232 taken along the intersecting direction, the depth D of the groove portion 232 is longer than the maximum outer diameter of each of the first elastic member 14a and the second elastic member 14b which have contracted after the cutting step. By making the groove portion 232 have such a sufficient depth, the first elastic member 14a and the second elastic member 14b are less likely to come out of the groove portion 232, making it possible to carry out the joining step without cutting the elastic members 14a and 14b. The measurement methods for the first elastic member 14a and the second elastic member 14b when fully stretched and when contracted are the same as the above-described measurement method for the elastic member 14.

In addition, like the modified example shown in FIG. 11A, in cases of the groove portion 232 has a configuration where, in a cross section of the groove portion 232 taken along the intersecting direction, the area ratio occupied by the portion 232up corresponding to the pair of first side-wall portions 232iu is larger than the area ratio occupied by the portion 232dw corresponding to the pair of second side-wall portions 232id, even if the first elastic member 14a and the second elastic member 14b having different thicknesses in the stretched state are arranged, when passing the thick second elastic member 14b through the groove portion 232 while receiving the elastic members 14a and 14b on the starting edge 232s side, adjustments can be made on the far side (232d2 side) of the groove portion 232. This makes it possible to carry out the joining step more stably.

Next, the above-mentioned first elastic member 14a and second elastic member 14b will be described in relation to the elastic members 5 provided on the diaper 1 (FIGS. 4A and 4B). In the diaper 1 using the stretchy sheet 10 formed by the manufacturing method of the present embodiment (FIGS. 4A and 4B), a plurality of elastic members 5 that stretch/contract in the left-right direction of the diaper 1 are arranged side by side at intervals in the up-down direction in the front waist portion 3F and the back waist portion 3B, as described above. The positions, in the left-right direction, of the plurality of elastic members 5 with respect to the first sheet 11 and the second sheet 12 is restricted by the plurality of welded-portion pairs jP (not shown here) located on both sides, in the up-down direction, of the elastic members 5.

In addition, the plurality of elastic members 5 of the diaper 1 include a first elastic member 5a (corresponding to the second elastic member 14b) and a second elastic member 5b (corresponding to the first elastic member 14a) which have different thicknesses in a state where the diaper 1 (absorbent article) is maximally stretched in the left-right direction (see FIG. 4B). Similarly, the positions, in the left-right direction, of the first elastic member 5a and the second elastic member 5b with respect to the first sheet 11 the second sheet 12 are restricted by the plurality of welded-portion pairs jP (not shown here) located on both sides, in the up-down direction, of each of the first elastic member 5a and the second elastic member 5b. In the diaper 1 of the present embodiment, similar to the elastic member 14 described in FIG. 8B, in each of the first elastic member 5a and the second elastic member 5b, a damaged portion in which a part of the surface of each of the first elastic member 5a or the second elastic member 5b is damaged is not provided between the welded-portion pairs jP adjacent in the left-right direction.

By the first elastic member 5a and the second elastic member 5b, which have different thicknesses in a state where the diaper 1 (absorbent article) is maximally stretched in the left-right direction, being located between the first sheet 11 and the second sheet 12, it is possible to provide parts with different stretch factors even when using the elastic member 5 of the same thickness (yarn count). Furthermore, by arranging elastic members 5 having different thicknesses (yarn counts), such as a thin elastic member 5 having a high stretch factor, or a thick elastic member 5 having a low stretch factor, it is possible for each part of the diaper 1 to exhibit the desired stress or stretch characteristics.

Examples of the first elastic member 5a of the diaper 1 include an elastic string having a thickness of 940 dtex and a stretch factor of 2.7 times. Examples of the second elastic member 5b of the diaper 1 include an elastic string having a thickness of 470 dtex and a stretch factor of 2.3 times. Also, the elastic members 5 may include another elastic member different from the first elastic member 5a and the second elastic member 5b. For example, in order to make the diaper 1 fit more tightly around the waist, the plurality of elastic members 5 may include a plurality of lower elastic members that are wider in the stretched state than the first elastic member 5a, and that are located inside the second elastic member 5b in the longitudinal direction (below it in the up-down direction, see FIG. 4B). Furthermore, the plurality of elastic members 5 may include, for example, elastic members 5 having a different thickness and a different stretch factor, between the second elastic member 5b and the lower elastic member in the up-down direction. Example thereof include elastic strings having a thickness of 620 dtex and a stretch factor of 2.8 times, or elastic strings having a thickness of 780 dtex and a stretch factor of 2.7 times. In this way, by reliably placing multiple types of the elastic members 5 having different thicknesses and different stretch factors in the stretched state at the desired positions in the waist portion 3, it becomes easier to finely adjust the stress in the waist portion 3 to make it fit better around the waist of the wearer.

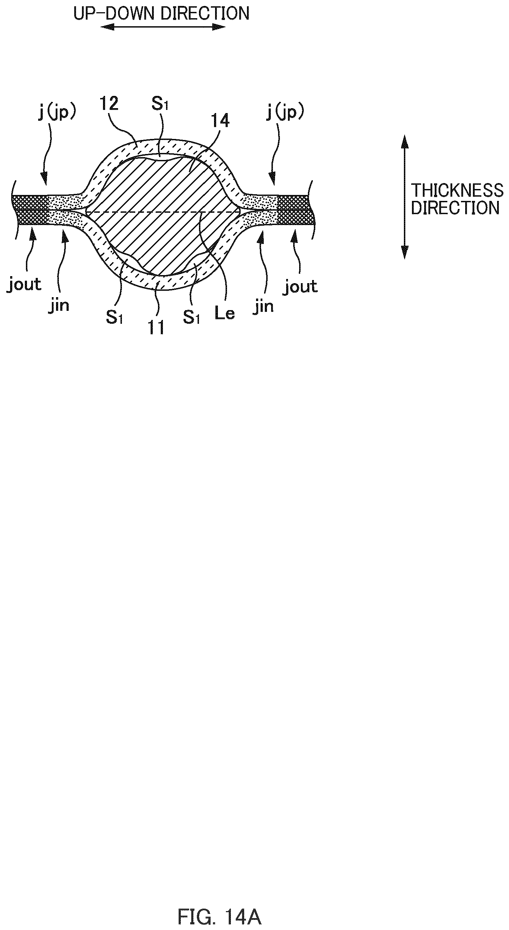

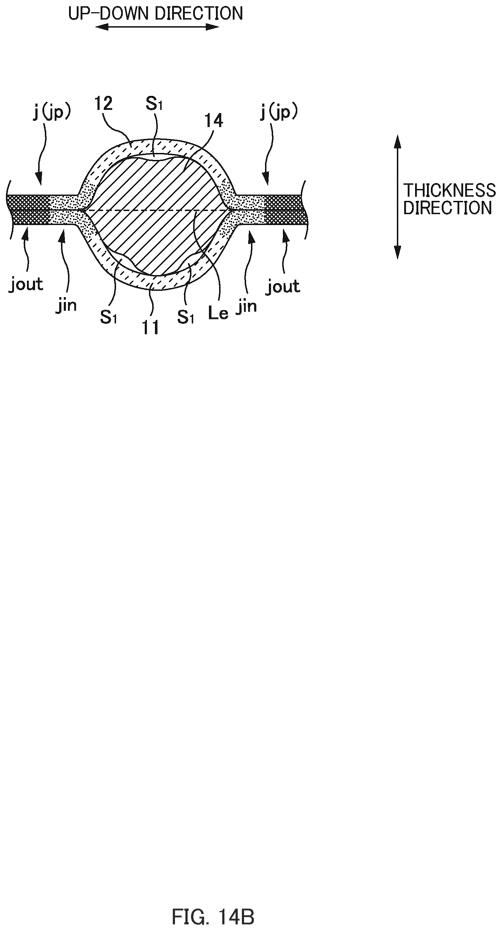

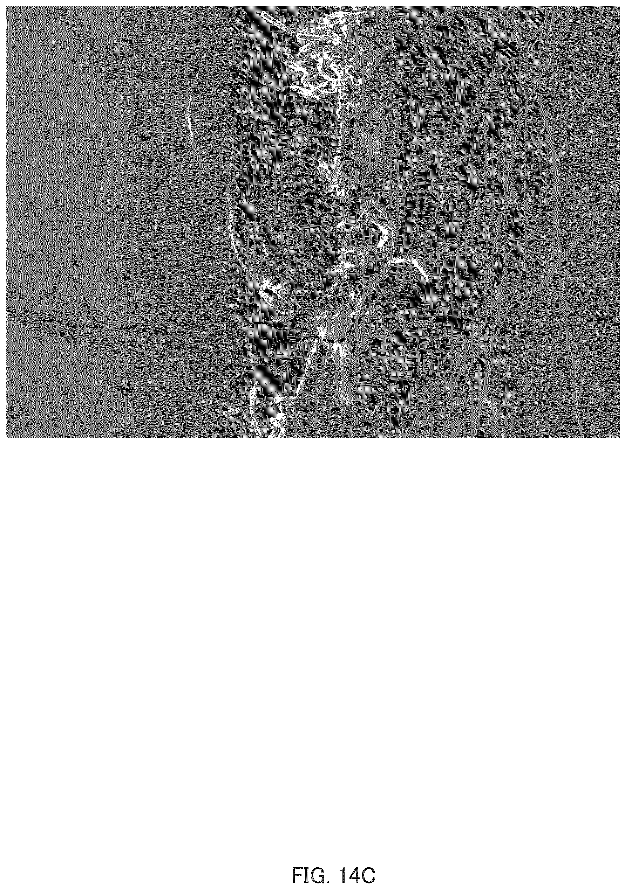

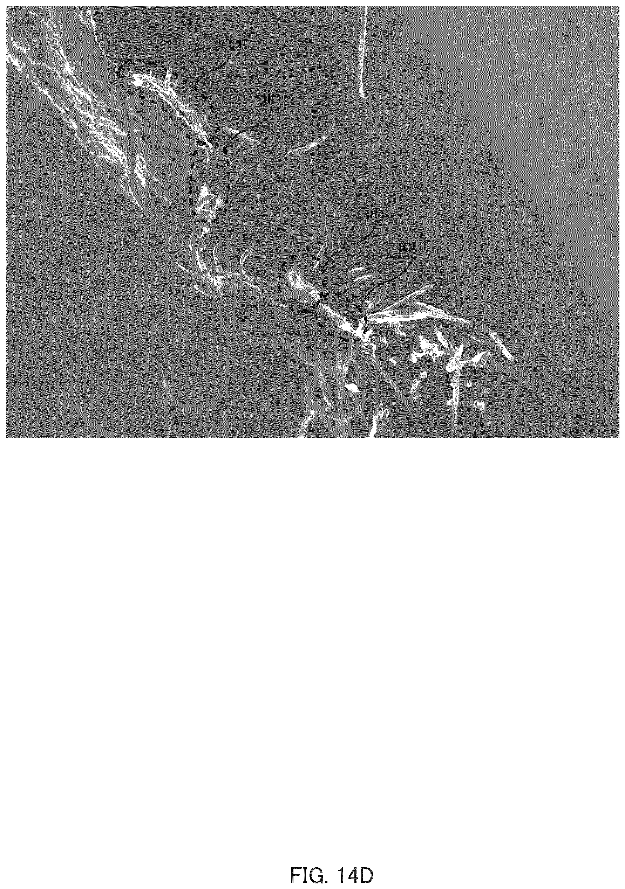

Further, the stretchy sheet 10 manufactured by the manufacturing method of the present embodiment further has a configuration described below. FIG. 14A is a schematic cross-sectional view of a portion of the stretchy sheet 10 where a welded-portion pair jP is formed, in the natural state (in a state in which the elastic member 14 is contracted). FIG. 14B is a schematic cross-sectional view of another example of a portion of the stretchy sheet 10 where the welded-portion pair jP is formed, in the natural state (in a state in which the elastic member 14 is contracted). FIG. 14C is a microscope image showing an example of an inner welded portion jin and an outer welded portion jout according to the present embodiment, and FIG. 14D is a microscope image showing another example of the inner welded portion jin and the outer welded portion jout according to the present embodiment.

As shown in FIG. 14A, the welded-portion pair jP formed in the stretchy sheet 10 manufactured by the manufacturing method of the present embodiment has the inner welded portion jin (the portion shown by the dot pattern in FIG. 14A) and the outer welded portion jout (the portion shown in FIG. 14A with a lattice pattern (so-called quilting pattern)). The inner welded portion jin includes fibers having melted-and-solidified portions in which the constituent fibers constituting the first sheet 11 and the second sheet 12 have been melted and solidified. The outer welded portion jout is located outside the inner welded portion jin in the up-down direction and includes only fibers having melted-and-solidified portions.

Specifically, the inner welded portion and the outer welded portion are formed as follows. Due to the manufacturing method of the stretchy sheet 10 and the configuration of the groove portions 232 according to the present embodiment, the welded portions j (welded-portion pairs jP) are formed without even the first elastic member 14a and the second elastic member 14b that have different thicknesses coming out of the groove portion 232. In the projection portion 231, the first sheet 11 and the second sheet 12 are firmly welded together, resulting in a thin thickness, and the outer welded portion jout is formed there, which includes only fibers having melted-and-solidified portions. In addition, the vibration of the horn 24 is transmitted to the first sheet 11 and the second sheet 12 which exist in the end portion of the groove portion 232 in the intersecting direction (up-down direction) from the end portion of the projection portion 231 in the intersecting direction (up-down direction), thereby forming the inner welded portion jin. The inner welded portion include a mixture of fibers having melted-and-solidified portions and fibers not having melted-and-solidified portions, or are entirely composed of fibers having melted-and-solidified portions. In the inner welded portion jin, the first sheet 11 and the second sheet 12 are melted and solidified more weakly than the outer welded portion jout, and the thickness of the inner welded portion jin is larger than the thickness of the outer welded portion jout.

More specifically, in the manufacturing method of the present embodiment, a stretchy sheet 10 $having the following feature is formed: in cases where the elastic member 14 is a thin elastic member 14, fibers having a melted-and-solidified portion (that is, to the inner welded portion jin) and fibers not having a melted-and-solidified portion exist together between the elastic member 14 and the outer welded portion jout; and in cases where the elastic member 14 is a thick elastic member 14, fibers having a melted-and-solidified portion (that is, to the inner welded portion jin) only exist between the elastic member 14 and the outer welded portion jout.

The cross-sectional condition of the stretchy sheet 10 in the natural state at the welded-portion pair jP can be confirmed by photographing a measurement sample which has been obtained by cutting the stretchy sheet 10 after the stretchy sheet 10 in the natural state being immersed in liquid nitrogen and solidified, using a scanning electron microscope (FlexSEM 1000, manufactured by Hitachi, Ltd.) (see FIGS. 14C and 14D). The inner welded portion jin shown in the lower part of FIG. 14C and the inner welded portion jin shown in the upper part of FIG. 14D show a state in which fibers having melted-and-solidified portions and fibers not having melted-and-solidified portions exist together. Furthermore, the inner welded portion jin shown in the upper part of FIG. 14C and the inner welded portion jin shown in the lower part of FIG. 14D show a state in which all of the fibers have melted-and-solidified portions. In the outer welded portion jout shown in FIGS. 14C and 14D, the fibers are all bundled together and integrated, and the outer welded portion jout part appears as a single mass.

In this way, by the welded portion j having the inner welded portion jin, the welded portion j is prevented from becoming too stiff, and in addition, even if force is applied to the inner welded portion jin and the positional restriction of the elastic member 14 weakens, the presence of the outer welded portion jout can prevent the elastic member 14 from coming out from between the first sheet 11 and the second sheet 12. In the present embodiment, both welded portions j, j of the welded-portion pair jP have the inner welded portion jin and the outer welded portion jout, but it is sufficient that at least one welded portion of the welded-portion pair has them.

Also, as mentioned above, the average thickness of the inner welded portion jin is thicker than the average thickness of the outer welded portion jout. Here, the "average thickness" refers to the average value obtained by measurements of multiple locations (for example, 10 locations) in each of the inner welded portion jin and the average value the outer welded portion jout, using a thickness gauge (the above-mentioned electron microscope). If the inner welded portion jin between which the elastic member 14 is sandwiched is thin, when the elastic member 14 contracts, there is a risk that a portion of the inner welded portion jin closest to the elastic member 14 (the inner end portion, in the up-down direction, of the inner welded portion jin) break and peeling easily occur from that point. If the force can be cushioned by the thickness of the inner welded portion, it is possible to prevent the outer welded portion from breaking or peeling off. Furthermore, even if the thick inner welded portion jin peels off, the presence of the outer welded portion jout allows the welded state to be maintained. Therefore, it is possible to prevent the elastic member 14 from passing through between the welded-portion pair jP with great force all at once (so-called coming off).

Furthermore, as shown in FIG. 14B, the inner welded portion jin may be formed to have a portion where it overlaps the elastic member 14 in the up-down direction, depending on the thickness of the elastic member 14 or the like. In the welded portion j shown in FIG. 14B, the inner welded portion jin is formed further inside in the up-down direction, compared to the inner welded portion jin shown in FIG. 14A. Even if the inner welded portion jin is formed so as to have an overlapping portion with the elastic member 14 in this manner, the cutting of the elastic member 14 is suppressed because the welding is weaker than that of the outer welded portion jout. In addition, by supporting both sides of the elastic member 14 in the up-down direction by thick parts (inner welded portion jin), the force can be cushioned.

Also, as shown in FIG. 14A, in a cross section taken along the up-down direction, the stretchy sheet 10 has a portion where the void S₁ is formed at least either between the elastic member 14 and the first sheet 11, or between the elastic member 14 and the second sheet 12. In cases of the stretchy sheet 10 manufactured by the manufacturing method of the present embodiment, the first sheet 11 and the second sheet 12 are joined in a state in which the elastic members 14 are stably positioned without coming out of the groove portions 232, and therefore when the elastic members 14 contract and the positions in the left-right direction are restricted, the elastic member 14 remains without excessive contact with the first sheet 11 and the second sheet 12, and the void S₁ is formed. In this way, by placing the elastic member 14 while forming the void S1, the elastic member 14 sandwiched between the welded-portion pair jP is prevented from becoming too stiff when the stretchy sheet 10 contracts.

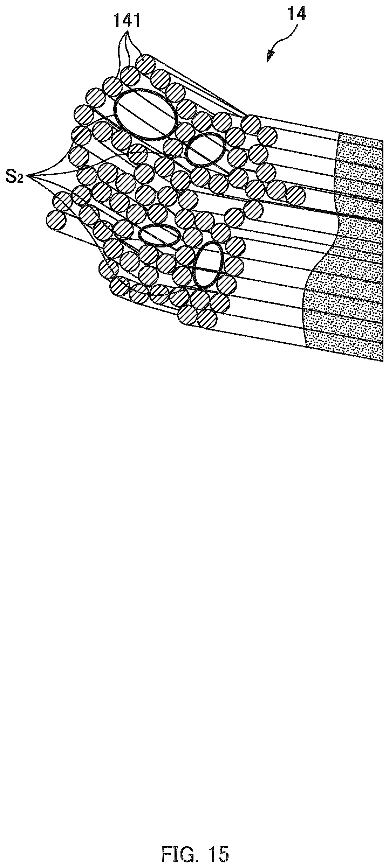

FIG. 15 is a diagram illustrating the structure of the elastic member 14. As described above, examples of the elastic member 14 used in the present embodiment include urethane-based rubber, ester-based rubber, latex (natural rubber), etc., but among these, it is preferable that the elastic member is a string-like material made of a plurality of elastic fibers 141 such as polyurethane or polyester, as shown in FIG. 15. In this case, the outer shape of the elastic member 14 becomes uneven, increasing the surface area that come into contact with the constituent fibers of the first sheet 11 and the second sheet 12, and increasing the frictional force with the sheet. Therefore, it is possible to easily prevent the elastic member 14 from coming off from among the welded-portion pairs jP.

In addition, in a cross section taken along the up-down direction between the welded-portion pair jP between which the elastic member 14 in a state of contracting in the left-right direction is sandwiched, the sandwiched portion of the elastic member 14 is thinner than the portion that is not sandwiched between the welded-portion pair jP, but the void S₂ may be formed between the elastic fibers of the sandwiched portion of the elastic member 14 (FIG. 15). By doing so, even if external pressure is applied to the elastic member 14, the void S₂ serves as an escape space by being compressed, making it difficult for the elastic member 14 to be cut off.

Furthermore, as shown in FIG. 14A, in the cross section, in the natural state, of a portion of the stretchy sheet 10 where the welded-portion pair jP, it is preferable that the ratio of the area on the one side of the elastic member 14 in the thickness direction from a line Le connecting both side portions, in the up-down direction, of the elastic member 14 to the area on the other side from the line Le is in the range of 20:80 to 80:20. In other words, the elastic member 14, whose position in the left-right direction is restricted by the welded-portion pair jP, is less offset from the center (less eccentric), making it easier to clamp when the elastic member 14 is cut, and even if force is applied to stretch the stretchy sheet 10 during use, the elastic member is less likely to come out of the welded-portion pair jP.

It is also preferable to use spandex as the elastic members 14, in which case it is desirable that the number of single yarns constituting the elastic member 14 is, for example, 50 or more, and that the titanium oxide content of the spandex is 3.0% or less by weight. By adopting such a spandex as the elastic members 14, the elastic members 14 are less likely to be cut.

### Others

The above embodiment is to facilitate understanding of the present disclosure and is not in any way to be construed as limiting the present disclosure. The present disclosure may variously be changed or altered without departing from its gist and encompass equivalents thereof.

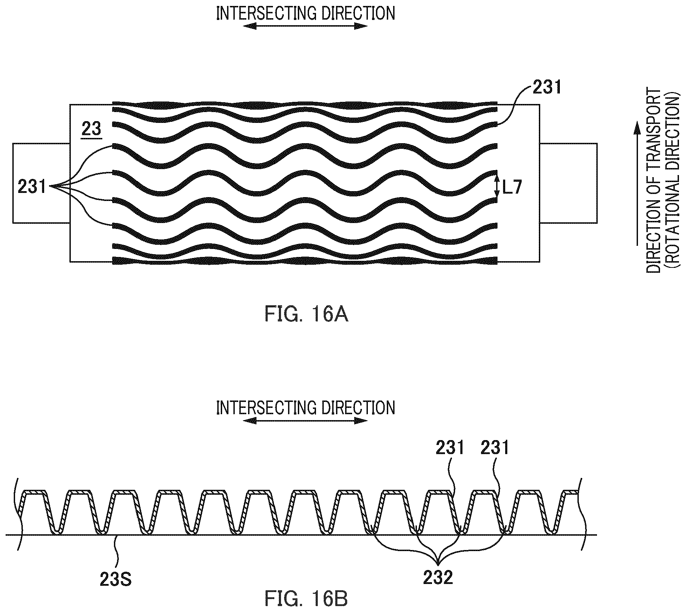

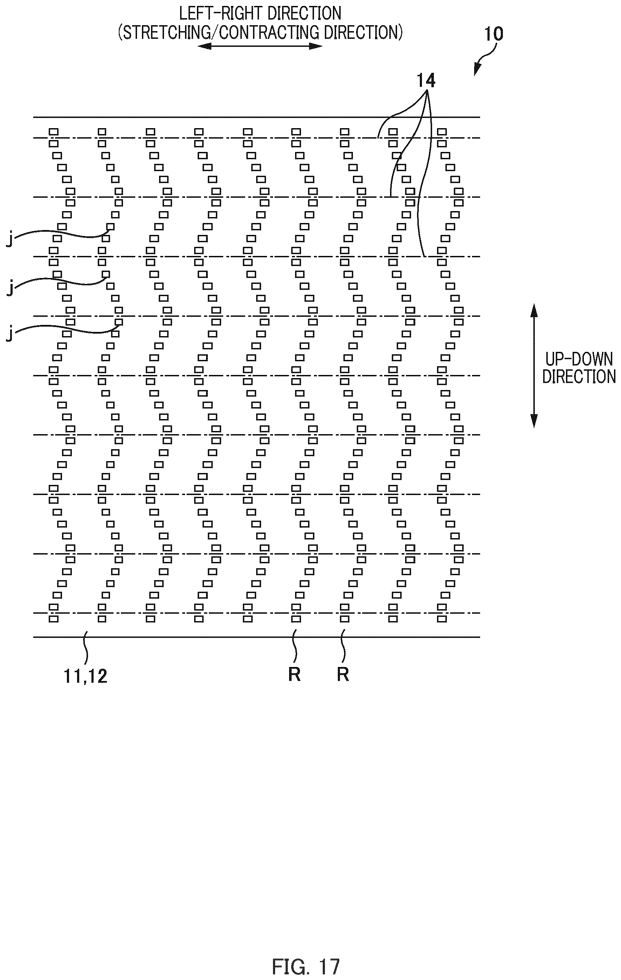

FIG. 16A shows a modified example of the anvil roll 23, and FIG. 16B is a diagram illustrating groove portions 232 of the anvil roll 23 in FIG. 16A. FIG. 17 shows a modified example of the stretchy sheet 10, and is a diagram of the stretchy sheet 10 in a state of being stretched to eliminate wrinkles. In the above embodiment, regarding the projection portions 231 provided in the anvil roll 23 used in the joining step, the projection portions 231 each having a specified number of the groove portions 232 (the projection portions 231 that form the welded-portion group jG of the stretchy sheet 10) are provided at intervals in the intersecting direction. However, the present invention is not limited to this. As shown in FIGS. 16A and 16B, the projection portions 231 of the anvil roll 23 may be located continuously in the intersecting direction. The rows of the projection portions 231 arranged continuously in the intersecting direction are arranged in plurality at specified intervals L7 in the rotational direction (direction of transport). In addition, in the anvil roll 23 of the modified example, the groove portions 232 may be provided continuously at intervals in the intersecting direction, as shown in FIG. 16B. In addition, the rows of the projection portions 231 extend along the intersecting direction (CD direction) while meandering in the rotational direction, but the present invention is not limited to this. The rows may extend in a straight manner in the intersecting direction (CD direction) without meandering in the rotational direction.

The stretchy sheet 10 shown in FIG. 17 is an example of the stretchy sheet 10 formed when the joining step described above is performed using the anvil roll 23 shown in FIGS. 16A and 16B. In the stretchy sheet 10 of the modified example, there are provided the welded-portion rows R each having a plurality of the welded portions j arranged at specified intervals in the up-down direction and the left-right direction. In addition, the plurality of welded-portion rows R are located at intervals in the left-right direction. As shown in FIG. 17, the welded-portion rows R in the modified example each extend in the up-down direction while meandering in the left-right direction, that is, the positions, in the left-right direction, of the welded portions j adjacent in the up-down direction are shifted. However, the welded-portion row R may be formed so as to extend along the up-down direction without meandering in the left-right direction.

### REFERENCE SIGNS LIST

1: Underpants-shaped disposable diaper (absorbent article);
2: Absorbent main body;
3: Pair of waist portion;
3B: Back waist portion;
3F: Front waist portion;
5: Elastic member;
5a: First elastic member;
5b: Second elastic member;
10: Stretchy sheet;
10': Stacked body;
11: First sheet;
12: Second sheet;
14: Elastic member;
14a: First elastic member;
14b: Second elastic member;
20: Manufacturing apparatus;
21: Transport roller group (supplying section);
22: Ultrasonic welding unit (welded-portion forming section);
23: Anvil roll;
23s: Outer circumferential surface;
24: Horn;
24h: Vibrating surface;
24s: Supporting member;
25: Discharging roll;
26: Cutter roll (cutting section);
27: Receiving roll (cutting section);
31: Nip roll;
32: Nip roll;
141: Elastic fiber;
211: First transport roller;
212: Second transport roller;
213: Third transport roller;
231: Projection portion;
232: Groove portion;
232b: Bottom portion;
232id: Second side-wall portion;
232iu: First side-wall portion;
232s: Starting edge;
261: Cutter blade;
271: Receiving blade.

## Claims

1. A method for manufacturing a stretchy sheet in which a first sheet and a second sheet are stacked with an elastic member in between,
the method comprising:
a step of supplying the first sheet, the second sheet, and the elastic member to an anvil roll that rotates in a direction of transport,
the elastic member being in a state of stretching in the direction of transport and being placed between the first sheet and the second sheet;
a joining step of joining the first sheet and the second sheet by forming a welded-portion pair on both sides, in an intersecting direction perpendicular to the direction of transport, of the elastic member; and
a step of cutting the elastic member,
wherein after the step of cutting, the elastic member that has contracted is sandwiched between the welded-portion pair, and a position, in the direction of transport, of the elastic member is restricted,
wherein an outer circumferential surface of the anvil roll has a plurality of projection portions that project outside in a radial direction of the anvil roll,
wherein the projection portions have a groove portion along a rotational direction of the anvil roll,
wherein, in a cross section of the groove portion, taken along the intersecting direction, the groove portion has
a pair of first side-wall portions extending from a starting edge of the groove portion to a specified position on a far side, and
a pair of second side-wall portions extending from the specified position to a bottom portion of the groove portion,
wherein, in the cross section, when intersection points of a line connecting starting edges of the pair of first side-wall portions in the intersecting direction and lines respectively extending the pair of second side-wall portions toward the starting edges are a first intersection point and a second intersection point,
a separation distance between the starting edges of the pair of first side-wall portions is longer than a distance between the first intersection point and the second intersection point.

2. A method for manufacturing a stretchy sheet in which a first sheet and a second sheet are stacked with an elastic member in between,
the method comprising:
a step of supplying the first sheet, the second sheet, and the elastic member to an anvil roll that rotates in a direction of transport,
the elastic member being in a state of stretching in the direction of transport and being placed between the first sheet and the second sheet;
a joining step of joining the first sheet and the second sheet by forming a welded-portion pair on both sides, in an intersecting direction perpendicular to the direction of transport, of the elastic member; and
a step of cutting the elastic member,
wherein after the step of cutting, the elastic member that has contracted is sandwiched between the welded-portion pair, and a position, in the direction of transport, of the elastic member is restricted,
wherein an outer circumferential surface of the anvil roll has a plurality of projection portions that project outside in a radial direction of the anvil roll,
wherein the projection portions have a groove portion along a rotational direction of the anvil roll,
wherein, in a cross section of the groove portion, taken along the intersecting direction,
a depth of the groove portion is equal to or greater than a height of the projection portion from the outer circumferential surface, and
a length, in the intersecting direction, of the groove portion is shorter from the starting edge of the groove portion toward a far side.

3. The manufacturing method of the stretchy sheet according to claim 1 or 2, wherein
the projection portions have a plurality of the groove portions for the elastic member,
the plurality of groove portions being located spaced apart in the intersecting direction.

4. The manufacturing method of the stretchy sheet according to claim 3, wherein
in the cross section, a depth of the groove portions is greater than or equal to a spacing of the groove portions in the intersecting direction.

5. The manufacturing method of the stretchy sheet according to claim 1, wherein
in the cross section, a length, in the intersecting direction, of the groove portion is shorter from the starting edge of the groove portion toward a far side.

6. The manufacturing method of the stretchy sheet according to claim 1, wherein
in the cross section, an angle formed by the pair of first side-wall portions is equal to or greater than twice an angle formed by the pair of second side-wall portions.

7. The manufacturing method of the stretchy sheet according to claim 1, wherein
in a depth direction of the groove portion in the cross section,
a length of a portion, of the groove portion, formed by the pair of second side-wall portions is longer than a length of a portion, of the groove portion, formed by the pair of first side-wall portions.

8. The manufacturing method of the stretchy sheet according to claim 1 or 2, wherein
a depth of the groove portion in the cross section is greater than an outer diameter of the elastic member when the elastic member is stretched to its maximum stretchable length in the direction of transport.

9. The manufacturing method of the stretchy sheet according to claim 8, wherein
a depth of the groove portion in the cross section is greater than a maximum outer diameter of the elastic member which has contracted after the cutting step.

10. The manufacturing method of the stretchy sheet according to claim 1, wherein
in the cross section, an area ratio occupied by a portion of the groove portion that corresponds to the pair of first side-wall portions, in the depth direction of the groove portion, is larger than an area ratio occupied by a portion of the groove portion that corresponds to the pair of second side-wall portions.

11. The manufacturing method of the stretchy sheet according to claim 2, wherein,
in a cross section of the groove portion, taken along the intersecting direction, the groove portion has
a pair of first side-wall portions extending from a starting edge of the groove portion to a specified position on a far side, and
a pair of second side-wall portions extending from the specified position to a bottom portion of the groove portion, and
in the cross section, when intersection points of a line connecting starting edges of the pair of first side-wall portions in the intersecting direction and lines respectively extending the pair of second side-wall portions toward the starting edges are a first intersection point and a second intersection point,
a separation distance between the starting edges of the pair of first side-wall portions is longer than a distance between the first intersection point and the second intersection point.

12. A method for manufacturing a stretchy sheet in which a first sheet and a second sheet are stacked with a plurality of elastic members in between,
the method comprising:
a step of supplying the first sheet, the second sheet, and the plurality of elastic members to an anvil roll that rotates in a direction of transport,
the plurality of elastic members being in a state of stretching in the direction of transport and a state of being placed between the first sheet and the second sheet, with spaced apart in an intersecting direction that is perpendicular to the direction of transport;
a joining step of joining the first sheet and the second sheet by forming a welded-portion pair on both sides, in the intersecting direction perpendicular to the direction of transport, of each of the elastic members; and
a step of cutting the elastic member,
wherein the plurality of elastic members supplied to the anvil roll in the supplying step include a first elastic member and a second elastic member having different thicknesses,
wherein, in the joining step, the welded-portion pair is formed for each of the first elastic member and the second elastic member so that a damaged portion in which a part of a surface is damaged is not formed, and
wherein in the joining step, the welded-portion pair is formed in such a manner that the first elastic member and the second elastic member that have contracted after the step of cutting are each sandwiched between the welded-portion pair corresponding to it so that positions, in the direction of transport, of the first elastic member and the second elastic member are restricted.

13. The manufacturing method of the stretchy sheet according to claim 12, wherein
an outer circumferential surface of the anvil roll has a plurality of projection portions that project outside in a radial direction of the anvil roll,
the plurality of projection portions have a groove portion along a rotational direction of the anvil roll, and
in the joining step, the welded-portion pair is formed without the first elastic member and the second elastic member coming out of the groove portions in which they are respectively placed.

14. The manufacturing method of the stretchy sheet according to claim 13, wherein
the projection portions have a plurality of the groove portions for a single one of the elastic members,
the plurality of groove portions being located spaced apart in the intersecting direction.

15. The manufacturing method of the stretchy sheet according to claim 14, wherein
in a cross section of the groove portion taken along the intersecting direction,
a depth of the groove portions is greater than or equal to a spacing of the groove portions in the intersecting direction.

16. The manufacturing method of the stretchy sheet according to claim 13 or 14, wherein
in a cross section of the groove portion taken along the intersecting direction,
a depth of the groove portion is greater than an outer diameter of each of the first elastic member and the second elastic member when each of the first elastic member and the second elastic member is stretched to its maximum stretchable length in the direction of transport.

17. The manufacturing method of the stretchy sheet according to claim 16, wherein
a depth of the groove portion in the cross section is greater than a maximum outer diameter of each of the first elastic member and the second elastic member which have contracted after the cutting step.

18. The manufacturing method of the stretchy sheet according to claim 12, wherein
an outer circumferential surface of the anvil roll has a plurality of projection portions that project outside in a radial direction of the anvil roll,
the projection portions have a groove portion along a rotational direction of the anvil roll,
in a cross section of the groove portion taken along the intersecting direction,
the groove portion has
a pair of first side-wall portions extending from a starting edge of the groove portion to a specified position on a far side, and
a pair of second side-wall portions extending from the specified position to a bottom portion of the groove portion, and
in the cross section, an area ratio occupied by a portion of the groove portion that corresponds to the pair of first side-wall portions, in the depth direction of the groove portion, is larger than an area ratio occupied by a portion of the groove portion that corresponds to the pair of second side-wall portions.

19. A stretchy sheet having an up-down direction and a left-right direction that intersect each other,
the stretchy sheet comprising:
a first sheet;
a second sheet that is joined to the first sheet by a plurality of welded portions; and
an elastic member that is provided between the first sheet and the second sheet and that is capable of stretching/contracting in the left-right direction,
wherein a plurality of welded-portion pairs are included
in which the plurality of welded portions are located on both sides, in the up-down direction, of the elastic member, and
that restricts a position, in the left-right direction, of the elastic member with respect to the first sheet and the second sheet, by sandwiching the elastic member in a state of being contracted in the left-right direction,
wherein, in a cross section of the stretchy sheet taken along the up-down direction at a formation position of the welded portion,
the welded-portion pair is provided on both sides, in the up-down direction, of the elastic member, and
at least either one welded portion of the welded-portion pair has
an inner welded portion that includes fibers having a melted-and-solidified portion in which constituent fibers constituting the first sheet and the second sheet have been melted and solidified, and
an outer welded portion that is located outside the inner welded portion in the up-down direction and that includes only fibers having the melted-and-solidified portion, and
wherein an average thickness of the inner welded portion is larger than an average thickness of the outer welded portion.

20. The stretchy sheet according to claim 19, wherein
in a cross section of the stretchy sheet taken along the up-down direction,
both welded portions of the welded-portion pair have the inner welded portion and the outer welded portion.

21. The stretchy sheet according to claim 19 or 20, wherein
in a cross section of the stretchy sheet taken along the up-down direction,
the stretchy sheet has a portion where a void is formed at least either between the elastic member and the first sheet or between the elastic member and the second sheet.

22. The stretchy sheet according to claim 19 or 20, wherein
the elastic member is a string-like elastic member made of a plurality of elastic fibers, and
the elastic member has a void between the elastic fibers, in a cross section taken along the up-down direction between the welded-portion pair between which the elastic member in a state of contracting in the left-right direction is sandwiched.

23. An absorbent article having an up-down direction and a left-right direction that intersect each other,
the absorbent article comprising:
an absorbent main body; and
a pair of waist portions,
wherein the absorbent article includes a first sheet, a second sheet that is joined to the first sheet by a plurality of welded portions, and an elastic member that is capable of stretching/contracting in the left-right direction,
wherein a plurality of the elastic members are located at intervals in the up-down direction between the first sheet and the second sheet,
wherein the plurality of welded portions includes a plurality of welded-portion pairs located on both sides, in the up-down direction, of the elastic member,
wherein the plurality of welded-portion pairs restricts a position, in the left-right direction, of the elastic member with respect to the first sheet and the second sheet, by sandwiching the elastic member in a state of being contracted in the left-right direction,
wherein the plurality of elastic members include a first elastic member and a second elastic member having different thicknesses in a state where the absorbent article is maximally stretched in the left-right direction, and
wherein, for each of the first elastic member and the second elastic member, a damaged portion in which a part of a surface of each of the first elastic member and the second elastic member is damaged is not provided between the welded-portion pairs adjacent in the left-right direction.

24. An apparatus for manufacturing a stretchy sheet in which is a first sheet and a second sheet are stacked with an elastic member in between,
the apparatus, comprising:
a supplying section that supplies the first sheet, the second sheet, and the elastic member to an anvil roll that rotates in a direction of transport,
the elastic member being in a state of stretching in the direction of transport and being placed between the first sheet and the second sheet;
a welded-portion forming section that forms a plurality of welded portions that join the first sheet and the second sheet, by forming a welded-portion pair on both sides, in an intersecting direction perpendicular to the direction of transport, of the elastic member; and
a cutting section that cuts the elastic member;
wherein after the step of cutting, the elastic member that has contracted is sandwiched between the welded-portion pair, and a position, in the direction of transport, of the elastic member is restricted,
wherein an outer circumferential surface of the anvil roll has a plurality of projection portions that project outside in a radial direction of the anvil roll,
wherein the projection portions have a groove portion along a rotational direction of the anvil roll,
wherein, in a cross section of the groove portion, taken along the intersecting direction, the groove portion has
a pair of first side-wall portions extending from a starting edge of the groove portion to a specified position on a far side, and
a pair of second side-wall portions extending from the specified position to a bottom portion of the groove portion,
wherein, in the cross section, when intersection points of a line connecting starting edges of the pair of first side-wall portions in the intersecting direction and lines respectively extending the pair of second side-wall portions toward the starting edges are a first intersection point and a second intersection point,
a separation distance between the starting edges of the pair of first side-wall portions is longer than a distance between the first intersection point and the second intersection point.

25. An apparatus for manufacturing a stretchy sheet in which is a first sheet and a second sheet are stacked with an elastic member in between,
the apparatus, comprising:
a supplying section that supplies the first sheet, the second sheet, and the elastic member to an anvil roll that rotates in a direction of transport,
the elastic member being in a state of stretching in the direction of transport and being placed between the first sheet and the second sheet;
a welded-portion forming section that forms a plurality of welded portions that join the first sheet and the second sheet, by forming a welded-portion pair on both sides, in an intersecting direction perpendicular to the direction of transport, of the elastic member; and
a cutting section that cuts the elastic member;
wherein after the step of cutting, the elastic member that has contracted is sandwiched between the welded-portion pair, and a position, in the direction of transport, of the elastic member is restricted,
wherein an outer circumferential surface of the anvil roll has a plurality of projection portions that project outside in a radial direction of the anvil roll,
wherein the projection portions have a groove portion along a rotational direction of the anvil roll,
wherein, in a cross section of the groove portion, taken along the intersecting direction,
a depth of the groove portion is equal to or greater than a height of the projection portion from the outer circumferential surface, and
a length, in the intersecting direction, of the groove portion is shorter from the starting edge of the groove portion toward a far side.

26. An apparatus for manufacturing a stretchy sheet in which a first sheet and a second sheet are stacked with a plurality of elastic members in between,
the apparatus comprising:
a supplying section that supplies the first sheet, the second sheet, and the plurality of elastic members to an anvil roll that rotates in a direction of transport,
the plurality of elastic members being in a state of stretching in the direction of transport and a state of being placed between the first sheet and the second sheet, with spaced apart in an intersecting direction that is perpendicular to the direction of transport;
a welded-portion forming section that forms a plurality of the welded portions that join the first sheet and the second sheet, by forming a welded-portion pair on both sides, in the intersecting direction perpendicular to the direction of transport, of each of the elastic members; and
a cutting section that cuts the elastic member,
wherein the plurality of elastic members supplied to the anvil roll in the supplying section include a first elastic member and a second elastic member having different thicknesses,
wherein, in the welded-portion forming section, the welded-portion pair is formed for each of the first elastic member and the second elastic member so that a damaged portion that a part of a surface is damaged is not formed, and
wherein in the welded-portion forming section, the welded-portion pair is formed in such a manner that the first elastic member and the second elastic member that have contracted after cutting by the cutting section are each sandwiched between the welded-portion pair corresponding to it so that positions, in the direction of transport, of the first elastic member and the second elastic member are restricted.

27. An anvil roll that uses for an apparatus for manufacturing a stretchy sheet in which a first sheet and a second sheet are stacked with the elastic member in between,
the anvil roll comprising: a plurality of projection portions that project outside in a radial direction of the anvil roll, on an outer circumferential surface of the anvil roll,
wherein the projection portions have a groove portion along a rotational direction of the anvil roll,
wherein, letting a direction perpendicular to the rotational direction be an intersecting direction, in a cross section of the groove portion taken along the intersecting direction,
the groove portion has
a pair of first side-wall portions extending from a starting edge of the groove portion to a specified position on a far side, and
a pair of second side-wall portions extending from the specified position to a bottom portion of the groove portion,
wherein, in the cross section, when intersection points of a line connecting starting edges of the pair of first side-wall portions in the intersecting direction and lines respectively extending the pair of second side-wall portions toward the starting edges are a first intersection point and a second intersection point,
a separation distance between the starting edges of the pair of first side-wall portions is longer than a distance between the first intersection point and the second intersection point.

28. An anvil roll that uses for an apparatus for manufacturing a stretchy sheet in which a first sheet and a second sheet are stacked with the elastic member in between,
the anvil roll comprising: a plurality of projection portions that project outside in a radial direction of the anvil roll, on an outer circumferential surface of the anvil roll,
wherein the projection portions have a groove portion along a rotational direction of the anvil roll,
wherein, letting a direction perpendicular to the rotational direction be an intersecting direction, in a cross section of the groove portion taken along the intersecting direction,
a depth of the groove portion is equal to or greater than a height of the projection portion from the outer circumferential surface, and
a length, in the intersecting direction, of the groove portion is shorter from the starting edge of the groove portion toward a far side.
